Europäisches Patentamt

European Patent Office

Office européen des brevets

(19)

(11) **EP 1 449 926 A1**

(12) **EUROPEAN PATENT APPLICATION**
published in accordance with Art. 158(3) EPC

(43) Date of publication:
25.08.2004 Bulletin 2004/35

(51) Int Cl.7: **C12Q 3/00**, G06F 19/00

(21) Application number: 02790727.8

(86) International application number:
**PCT/JP2002/013012**

(22) Date of filing: 12.12.2002

(87) International publication number:
**WO 2003/054235 (03.07.2003 Gazette 2003/27)**

(84) Designated Contracting States:
**CH DE FR GB LI**

(30) Priority: 20.12.2001 JP 2001388414

(71) Applicant: **Japan Science and Technology Agency**
**Kawaguchi-shi, Saitama 332-0012 (JP)**

(72) Inventor: **KITANO, Hiroaki**
**Kawagoe-shi, Saitama 350-0035 (JP)**

(74) Representative: **HOFFMANN - EITLE**
**Patent- und Rechtsanwälte**
**Arabellastrasse 4**
**81925 München (DE)**

(54) **METABOLISM CIRCUIT INFORMATION PROCESSING METHOD&comma; METABOLISM CIRCUIT INFORMATION PROCESSOR&comma; PROGRAM&comma; AND RECORDING MEDIUM**

(57)    An apparatus according to this invention allows a user to set at least one of desired metabolic circuit information, environmental factor information, and end condition information, changes all of nodes and edges included in the metabolic circuit information or either the nodes or the edges based on at least one of the metabolic circuit information set by simulation condition setting and the environmental factor information until the end condition information set by the simulation condition setting is satisfied, executes a metabolic simulation by a metabolic flux balance analysis, and outputs a result of the simulation executed by simulation execution.

EXPLANATORY VIEW FOR PRINCIPLE OF PRESENT INVENTION

FIG.1

**Description**

TECHNICAL FIELD

The present invention relates to a metabolic circuit information processing method, a metabolic circuit information processing apparatus, a program, and a recording medium. More specifically, the present invention relates to a metabolic circuit information processing method, a metabolic circuit information processing apparatus, a program, and a recording medium that can simulate a metabolic circuit by a metabolic flux balance analysis.

BACKGROUND ART

In recent years, studies in analysis of a metabolic circuit for controlling metabolism such as fermentation are developing. The analysis of the metabolic circuit is intended to clarify what behavior a complex circuit exhibits and what functional properties the circuit possesses under various conditions. To attempt in-depth analysis of these properties, however, it is necessary to measure the structure of the metabolic circuit and constants (hereinafter, "reaction constants") related to respective reactions and the like. In respect of metabolic circuits, though limited to metabolism of partial model organisms such as colon bacilli and yeasts, metabolic circuit information databases such as EcoCyc and KEGG are constructed. However, it is not easy to measure reaction constants of respective metabolic reactions, and information stored in the databases is insufficient. Therefore, it is quite significant to develop a method of obtaining knowledge as much as possible only based on the structure of a metabolic reaction circuit without using information on the reaction constants.

A metabolic analysis has been proposed by Kacser & Burns (Kacser, H. and Burns, J., Genetics, 97, 639-666, 1981), Heinrich & Rapoport (Heinrich, R. and Rapoport, T., Eur. J. Biochem., 42, 89-95, 1974), and the others, and developed by researchers such as Fell (Fell, D.: Understanding the Control of Metabolism, Portland Press, London, 1996, Fell, D. Biochem. J., 286, 313-330, 1992).

The metabolic flux balance analysis ("FBA") is an approach belonging to the study of this metabolic analysis. In the metabolic FBA, even if constants related to metabolism cannot be measured sufficiently, a behavioral range and properties of a target metabolic circuit are analyzed based on fundamental constraint conditions such as the law of conservation of mass while using only structures of metabolic reactions.

A basic approach for the FBA made by Palsson et al., for example, is to describe metabolic reactions as simultaneous linear equations, and to define a vector space of a solution of the simultaneous equations. This vector space is transformed into a biochemically important basis, and a metabolic state for maximizing an objective function given by linear programming is finally specified (Shilling, C. and Palsson, B., Proc. Nat. Sci. Acad., 95, 4193-4198, 1998).

This FBA basic approach is applied to Haemophilus influenzae Rd (Edwards, J. and Palsson, B., Journal of Biological Chemistry, 274, 25, 17410-17416, 1999), E. coli K-12 (Edwards, J. and Palsson, B., BMC Bioinformatics, 1, 1, 2000, Edwards, J. and Palsson, B., Biotechnology and bioengineering, 58, Nos. 2 & 3, 162-169, 1998) and MG1655 (Edwards, J., Ibarra, R. and Palsson, B., Nature Biotechnology, 19, 125-130, 2001), human red blood cells (Jamshidi, N., et al. Bioinformatics, 3, 286-287, 2001), and the like, for carrying out metabolic circuit analysis.

Nevertheless, these conventional approaches have the following disadvantage. Although the information on stationary-state metabolic circuits stored in the metabolic circuit information databases can be acquired, information on a new metabolic circuit cannot be acquired.

In addition, these conventional approaches are disadvantageously incapable of acquiring information on an optimum metabolic circuit based on the balance between a metabolic efficiency and a metabolic cost or the like. Namely, none of the conventional approaches carry out a simulation by changing the information on the known stationary-state metabolic circuits, and make selection or the like of an optimum metabolic circuit which may possibly fulfill a user's objective.

Specifically, the FBA approach made by Palsson et al. is excellent in that only if the structure of the metabolic circuit is known from the metabolic circuit information databases or the like, many properties of the metabolic circuit can be identified based on the information on stationary-state metabolic circuits stored in the metabolic circuit information databases without the need of individual reaction constants. This approach has, however, a constraint that only the stationary-state metabolic circuits can be analyzed.

Furthermore, from viewpoints of industrial applicability, conditions that should be actually considered, for example, information on a culture environment and the like such as culture time and cost are not at all taken into consideration in the conventional FBA approaches. In addition, none of the conventional FBA approaches seiect the optimum metabolic circuit based on the balance between the metabolic efficiency and the cost or the like while using the information on the culture environment in the analysis. Consequently, it is difficult to apply analysis results obtained by the conventional approaches to actual experimental operations and production of metabolisms which are actual objectives of users.

In other words, none of the conventional approaches analyze a desired metabolic circuit in light of various environmental factors that have an effect on metabolism, the culture time, the cost, and the like, and acquire new metabolic circuit information which may possibly fulfill user's objective.

The conventional approaches for the metabolic circuit analysis have thus many disadvantages. As a result, the conventional approaches are inferior in convenience of metabolic information processing result and implementation efficiency for users who are to analyze metabolic information.

It is, therefore, an object of the present invention to provide a metabolic circuit information processing method, a metabolic circuit information processing apparatus, a program, and a recording medium that can acquire both of or one of information on a new metabolic circuit and information on a target optimum metabolic circuit.

DISCLOSURE OF THE INVENTION

One aspect of the present invention provides the metabolic circuit information processing method including: a metabolic circuit information setting step of allowing a user to set target metabolic circuit information that includes information on nodes respectively representing basic constituent elements of a metabolism and edges respectively representing reaction relationships among the basic constituent elements; an environmental factor information setting step of allowing the user to set environmental factor information on an environmental factor that influences both of or one of the basic constituent elements and the reaction relationships; an end condition information setting step of allowing the user to set end condition information on a condition for finishing a simulation; a simulation execution step of changing both of or one of one of the nodes and one of the edges included in the target metabolic circuit information, and of executing a metabolic simulation by a metabolic flux balance analysis using the environmental factor information set at the environmental factor information setting step; a simulation result determination step of determining whether a result of the simulation executed at the simulation execution step satisfies the end condition information set at the end condition information setting step, and of, if the result of the simulation does not satisfy the end condition information, changing both of or one of another one of the nodes and another one of the edges, followed by execution of the simulation execution step; and a simulation result output step of outputting the result of the simulation executed at the simulation execution step.

The method according to one aspect of the present invention allows a user to set target metabolic circuit information that includes information on nodes respectively representing basic constituent elements of a metabolism and edges respectively representing reaction relationships among the basic constituent elements; allows the user to set environmental factor information on an environmental factor that influences both of or one of the basic constituent elements and the reaction relationships; allows the user to set end condition information on a condition for finishing a simulation; changes both of or one of one of the nodes and one of the edges included in the target metabolic circuit information, and executes a metabolic simulation by a metabolic flux balance analysis using the set environmental factor information; determines whether a result of the simulation satisfies the set end condition information, and, if the result of the simulation does not satisfy the end condition information, changes both of or one of another one of the nodes and another one of the edges, followed by execution of the simulation execution step; and outputs the result of the executed simulation. Therefore, the user can easily set various conditions according to a user's objective, and a user's target optimum metabolic circuit information or new metabolic circuit information can be acquired by performing a simulation processing. By user's setting, for example, a toxic matter such as dioxin or a persistent matter such as plastic as a substrate (starting material) and obtaining information on an optimum metabolic circuit for metabolizing the substrate, the information can be used for biodegradation studies on toxic matters and persistent matters. In addition, if the user sets an intermediate metabolite or a final metabolite such as a low-molecular compound, a sugar, a protein, or an amino acid, then optimum metabolic circuit information for producing metabolites can be acquired from a simulation result, and pharmaceuticals and foods can be produced using this metabolic circuit information. Furthermore, by setting a matter in vivo (e.g., a sugar, a protein, an amino acid, a DNA, an RNA, or a signal transmitter) and acquiring the metabolic circuit information, knowledge related to a disease or the like caused by a metabolic disorder resulting from the matter in vivo can be acquired.

Another aspect of the present invention provides the metabolic circuit information processing method according to the above aspect, wherein each of the basic constituent elements includes information on at least one of a substrate, a metabolite, and a matter in vivo.

This specifically depicts one example of the basic constituent elements. According to the method, each of the basic constituent elements includes information on at least one of a substrate, a metabolite, and a matter in vivo. Therefore, if the user sets an intermediate metabolite or a final metabolite such as a low-molecular compound, a sugar, a protein, or an amino acid produced by microorganisms, pharmaceuticals, pharmaceutical intermediates, alcohols, amino acids, or the like can be produced with high efficiency by acquiring optimum metabolic circuit information. Furthermore, by setting a matter in vivo (e.g., a sugar, a protein, an amino acid, a DNA, an RNA, or a signal transmitter), new metabolic circuit information related to various diseases and the like can be acquired from the simulation result.

Still another aspect of the present invention provides the metabolic circuit information processing method according to the above aspect, wherein the reaction relationships includes information on a relationship of at least one of an enzyme reaction, a transcription control reaction, a translation control reaction, and a chemical reaction between the basic constituent elements.

This specifically depicts one example of the reaction relationships. According to the method, each of the reaction relationships includes information on a relationship of at least one of an enzyme reaction, a transcription control reaction, a translation control reaction, and a chemical reaction between the basic constituent elements. Therefore, in relation to the enzyme reaction, for example, information on a synthetic rate and a decomposition rate for the enzyme reaction between a substrate and a metabolite can be defined as edge information, and simulation can be carried out based on the information. In relation to the transcription control reaction, information such as a transcription rate, a transcription promoter, and a transcription inhibitor for the transcription control reaction between a DNA and an RNA can be defined as edge information, and simulation can be carried out based on the information. In relation to the translation control reaction, a translation rate, a translation promoter, and a translation inhibitor for the translation control reaction between an mRNA and a protein can be defined as edge information, and simulation can be carried out based on the information. In relation to the chemical reaction, information such as a structural change, a polymerization property, a stability, and a chemical equilibrium for the chemical reaction between metabolites can be defined as edge information, and simulation can be carried out based on the information.

Still another aspect of the present invention provides the metabolic circuit information processing method according to the above aspect, wherein the environmental factor information includes information on at least one of a temperature, a pH, an atmospheric pressure, the number of times of stirring, a dissolved oxygen quantity, a culture medium component, an optical condition, and a carbon dioxide concentration.

This specifically depicts one example of the environmental factor information. According to the method, the environmental factor information includes information on at least one of a temperature, a pH, an atmospheric pressure, the number of times of stirring, a dissolved oxygen quantity, a culture medium component, an optical condition, and a carbon dioxide concentration. Therefore, simulation can be carried out based on information on an upper limit and a lower limit of a temperature, a pH, an atmospheric pressure, the number of times of stirring, a dissolved oxygen quantity, culture medium components, optical conditions, a carbon dioxide concentration, or the like that is feasible for an operation in a laboratory or a factory.

Still another aspect of the present invention provides the metabolic circuit information processing method according to the above aspect, wherein the end condition information includes information on at least one of a culture time, a metabolic efficiency, a metabolite quantity, the number of times of simulation, a cell density, a culture cost, a pH, a dissolved oxygen quantity, and a culture medium component quantity.

This specifically depicts one example of the end condition information. According to the method, the end condition information includes information on at least one of a culture time, a metabolic efficiency, a metabolite quantity, the number of times of simulation, a cell density, a culture cost, a pH, a dissolved oxygen quantity, and a culture medium component quantity. Therefore, if the culture time and the culture cost, for example, are set as the end condition information, the user can know an optimum simulation result within user's desired ranges of the culture time and the culture cost and thus, metabolic circuit information usable for actual production can be acquired. In addition, if the metabolic efficiency and the metabolite quantity are set as the end condition information, then simulation can be carried out until the metabolic efficiency and the metabolite quantity reach a target efficiency and a target quantity, respectively, and metabolic circuit information such as culture medium components and culture time necessary to attain the target metabolic efficiency and the target metabolite quantity can be acquired. If the cell density, the pH, the dissolved oxygen quantity, and the culture medium component quantity are set as the end condition information, then simulation can be carried out until they reach a set pH, a set culture medium component quantity, and the like, respectively, and metabolic circuit information in light of a change in a culture state can be acquired. If the number of times of simulation is set as the end condition information, optimum metabolic circuit information can be acquired by executing simulation by a certain number of times. Therefore, the disadvantage in that it takes considerable long calculation time to acquire the metabolic circuit information due to a great calculation quantity can be avoided.

The metabolic circuit information processing method according to still another aspect of the present invention further includes: a mutation frequency information storage step of storing mutation frequency information on a frequency with which both of or one of the one node and the one edge included in the metabolic circuit information mutates to both of or one of another one of the nodes and another one of the edges, wherein at the simulation execution step, both of or one of the one node and the one edge included in the metabolic circuit information is changed using the mutation frequency information stored at the mutation frequency information storage step.

According to the method, mutation frequency information on a frequency with which both of or one of the one node and the one edge included in the metabolic circuit information mutates to both of or one of another one of the nodes and another one of the edges is stored. Both of or one of the one node and the one edge included in the metabolic circuit information is changed using the stored mutation frequency information. Therefore, the metabolic circuit information can be efficiently changed.

Still another aspect of the present invention provides the metabolic circuit information processing method according to the above aspect, wherein, at the simulation execution step, both of or one of the one node and the one edge included in the metabolic circuit information is changed using at least one of a genetic algorithm and simulated annealing.

According to the method, both of or one of the one node and the one edge included in the metabolic circuit information is changed using at least one of a genetic algorithm and simulated annealing. Therefore, by applying a known optimization algorithm, the user can efficiently acquire optimum new metabolic circuit information other than stationary-state metabolic circuit information.

The present invention relates to a metabolic circuit information processing apparatus. Still another aspect of the present invention provides the metabolic circuit information processing apparatus including: a metabolic circuit information storage unit that stores metabolic circuit information including information on nodes respectively representing basic constituent elements of a metabolism and edges respectively representing reaction relationships among the basic constituent elements; an environmental factor information storage unit that stores environmental factor information on an environmental factor that influences both of or one of the basic constituent elements and the reaction relationships; an end condition information storage unit that stores end condition information on condition for finishing a simulation; a simulation condition setting unit that allows a user to set at least one of the metabolic circuit information, the environmental factor information, and the end condition information desired by the user; a simulation execution unit that changes both of or one of one of the nodes and one of the edges included in the metabolic circuit information, and executes a metabolic simulation by a metabolic flux balance analysis based on at least one of the metabolic circuit information set by simulation condition setting unit and the environmental factor information until the end condition information set by the simulation condition setting unit is satisfied; and a simulation result output unit that outputs a result of the simulation executed by the simulation execution unit.

The apparatus according to still another aspect of the present invention stores metabolic circuit information including information on nodes respectively representing basic constituent elements of a metabolism and edges respectively representing reaction relationships among the basic constituent elements; stores end condition information on a condition for finishing a simulation; stores environmental factor information on an environmental factor that influences both of or one of the basic constituent elements and the reaction relationships; allows a user to set at least one of the metabolic circuit information, the environmental factor information, and the end condition information desired by the user; changes both of or one of one of the nodes and one of the edges included in the metabolic circuit information, and executes a metabolic simulation by a metabolic flux balance analysis based on at least one of the set metabolic circuit information and the environmental factor information until the set end condition information is satisfied; and outputs a result of the executed metabolic simulation.

Therefore, the user can easily set various conditions according to a user's objective, and a user's target optimum metabolic circuit information or new metabolic circuit information can be acquired by performing a simulation processing. By user's setting, for example, a toxic matter such as dioxin or a persistent matter such as plastic as a substrate (starting material) and obtaining information on an optimum metabolic circuit for metabolizing the substrate, the information can be used for biodegradation of toxic matters and persistent matters. In addition, if the user sets an intermediate metabolite or a final metabolite such as a low-molecular compound, a sugar, a protein, or an amino acid, then optimum metabolic circuit information for producing metabolites can be acquired from a simulation result, and pharmaceuticals and foods can be produced using this metabolic circuit information. Furthermore, by setting a matter in vivo (e.g., a sugar, a protein, an amino acid, a DNA, an RNA, or a signal transmitter) and acquiring the metabolic circuit information, knowledge related to a disease or the like caused by a metabolic disorder resulting from the matter in vivo can be acquired.

Still another aspect of the present invention provides the metabolic circuit information processing apparatus according to the above aspect, wherein each of the basic constituent elements includes information on at least one of a substrate, a metabolite, and a matter in vivo.

This specifically depicts one example of the basic constituent elements. According to the apparatus, each of the basic constituent elements includes information on at least one of a substrate, a metabolite, and a matter in vivo. Therefore, if the user sets an intermediate metabolite or a final metabolite such as a low-molecular compound, a sugar, a protein, or an amino acid produced by microorganisms, pharmaceuticals, pharmaceutical intermediates, alcohols, amino acids, or the like can be produced with high efficiency by acquiring optimum metabolic circuit information. Furthermore, by setting a matter in vivo (e.g., a sugar, a protein, an amino acid, a DNA, an RNA, or a signal transmitter), new metabolic circuit information related to various diseases and the like can be acquired from the simulation result.

Still another aspect of the present invention provides the metabolic circuit information processing apparatus according to the above aspect, wherein each of the reaction relationships includes information on a relationship of at least one of an enzyme reaction, a transcription control reaction, a translation control reaction, and a chemical reaction between the basic constituent elements.

This specifically depicts one example of the reaction relationships. According to the apparatus, each of the reaction relationships includes information on a relationship of at least one of an enzyme reaction, a transcription control reaction, a translation control reaction, and a chemical reaction between the basic constituent elements. Therefore, in relation to the enzyme reaction, for example, information on a synthetic rate and a decomposition rate for the enzyme reaction between a substrate and a metabolite can be defined as edge information, and simulation can be carried out based on the information. In relation to the transcription control reaction, information such as a transcription rate, a transcription promoter, and a transcription inhibitor for the transcription control reaction between a DNA and an RNA can be defined as edge information, and simulation can be carried out based on the information. In relation to the translation control reaction, a translation rate, a translation promoter, and a translation inhibitor for the translation control reaction between an mRNA and a protein can be defined as edge information, and simulation can be carried out based on the information. In relation to the chemical reaction, information such as a structural change, a polymerization property, a stability, and a chemical equilibrium for the chemical reaction between metabolites can be defined as edge information, and simulation can be carried out based on the information.

Still another aspect of the present invention provides the metabolic circuit information processing apparatus according to the above aspect, wherein the environmental factor information includes information on at least one of a temperature, a pH, an atmospheric pressure, the number of times of stirring, a dissolved oxygen quantity, a culture medium component, an optical condition, and a carbon dioxide concentration.

This specifically depicts one example of the environmental factor information. According to the apparatus, the environmental factor information includes information on at least one of a temperature, a pH, an atmospheric pressure, the number of times of stirring, a dissolved oxygen quantity, a culture medium component, an optical condition, and a carbon dioxide concentration. Therefore, simulation can be carried out based on information on an upper limit and a lower limit of a temperature, a pH, an atmospheric pressure, the number of times of stirring, a dissolved oxygen quantity, culture medium components, optical conditions, a carbon dioxide concentration, or the like that is feasible for an operation in a laboratory or a factory.

Still another aspect of the present invention provides the metabolic circuit information processing apparatus according to the above aspect, wherein the end condition information includes information on at least one of a culture time, a metabolic efficiency, a metabolite quantity, the number of times of simulation, a cell density, a culture cost, a pH, a dissolved oxygen quantity, and a culture medium component quantity.

This specifically depicts one example of the end condition information. According to the apparatus, the end condition information includes information on at least one of a culture time, a metabolic efficiency, a metabolite quantity, the number of times of simulation, a cell density, a culture cost, a pH, a dissolved oxygen quantity, and a culture medium component quantity. Therefore, if the culture time and the culture cost, for example, are set as the end condition information, the user can know an optimum simulation result within user's desired ranges of the culture time and the culture cost and thus, metabolic circuit information usable for actual production can be acquired. In addition, if the metabolic efficiency and the metabolite quantity are set as the end condition information, then simulation can be carried out until the metabolic efficiency and the metabolite quantity reach a target efficiency and a target quantity, respectively, and metabolic circuit information such as culture medium components and culture time necessary to attain the target metabolic efficiency and the target metabolite quantity can be acquired. If the cell density, the pH, the dissolved oxygen quantity, and the culture medium component quantity are set as the end condition information, then simulation can be carried out until they reach a set pH, a set culture medium component quantity, and the like, respectively, and metabolic circuit information in light of a change in a culture state can be acquired. If the number of times of simulation is set as the end condition information, optimum metabolic circuit information can be acquired by executing simulation by a certain number of times. Therefore, the disadvantage in that it takes considerable long calculation time to acquire the metabolic circuit information due to a great calculation quantity can be avoided.

The metabolic circuit information processing apparatus according to still another aspect of the present invention further includes: a mutation frequency information storage unit that stores mutation frequency information on a frequency with which both of or one of the one node and the one edge included in the metabolic circuit information mutates to both of or one of another one of the nodes and another one of the edges, wherein the simulation execution unit changes both of or one of the one node and the one edge included in the metabolic circuit information using the mutation frequency information stored by the mutation frequency information storage unit.

This specifically depicts one example of the simulation execution unit. According to the apparatus, mutation frequency information on a frequency with which both of or one of the one node and the one edge included in the metabolic circuit information mutates to both of or one of another one of the nodes and another one of the edges is stored. Both of or one of the one node and the one edge included in the metabolic circuit information is changed using the stored mutation frequency information. Therefore, the metabolic circuit information can be efficiently changed.

Still another aspect of the present invention provides the metabolic circuit information processing apparatus according to the above aspect, wherein the simulation execution unit changes both of or one of the one node and the one edge included in the metabolic circuit information using at least one of a genetic algorithm and simulated annealing.

This specifically depicts one example of the simulation execution unit. According to the apparatus, both of or one of the one node and the one edge included in the metabolic circuit information is changed using at least one of a genetic algorithm and simulated annealing. Therefore, by applying a known optimization algorithm, the user can efficiently acquire optimum new metabolic circuit information other than stationary-state metabolic circuit information.

The present invention relates to a program. Still another aspect of the present invention provides the program for allowing a computer to execute a metabolic circuit information processing method, including: a metabolic circuit information setting step of allowing a user to set target metabolic circuit information that includes information on nodes respectively representing basic constituent elements of a metabolism and edges respectively representing reaction relationships between the basic constituent elements; an environmental factor information setting step of allowing the user to set environmental factor information on an environmental factor that influences both of or one of the basic constituent elements and the reaction relationships; an end condition information setting step of allowing the user to set end condition information on a condition for finishing a simulation; a simulation execution step of changing both of or one of one of the nodes and one of the edges included in the target metabolic circuit information, and of executing a metabolic simulation by a metabolic flux balance analysis using the environmental factor information set at the environmental factor information setting step; a simulation result determination step of determining whether a result of the simulation executed at the simulation execution step satisfies the end condition information set at the end condition information setting step, and of, if the result of the simulation does not satisfy the end condition information, changing both of or one of another one of the nodes and another one of the edges, followed by execution of the simulation execution step; and a simulation result output step of outputting the result of the simulation executed at the simulation execution step.

The program according to still another aspect of the present invention allows a user to set target metabolic circuit information that includes information on nodes respectively representing basic constituent elements of a metabolism and edges respectively representing reaction relationships among the basic constituent elements; allows the user to set environmental factor information on an environmental factor that influences both of or one of the basic constituent elements and the reaction relationships; allows the user to set end condition information on a condition for finishing a simulation; changes both of or one of one of the nodes and one of the edges included in the target metabolic circuit information, and executes a metabolic simulation by a metabolic flux balance analysis using the set environmental factor information; determines whether a result of the simulation satisfies the set end condition information, and, if the result of the simulation does not satisfy the end condition information, changes both of or one of another one of the nodes and another one of the edges; and outputs the result of the executed simulation. Therefore, the user can easily set various conditions according to a user's objective, and a user's target optimum metabolic circuit information or new metabolic circuit information can be acquired by performing a simulation processing. By user's setting, for example, a toxic matter such as dioxin or a persistent matter such as plastic as a substrate (starting material) and obtaining information on an optimum metabolic circuit for metabolizing the substrate, the information can be used for biodegradation of toxic matters and persistent matters. In addition, if the user sets an intermediate metabolite or a final metabolite such as a low-molecular compound, a sugar, a protein, or an amino acid, then optimum metabolic circuit information for producing metabolites can be acquired from a simulation result, and pharmaceuticals and foods can be produced using this metabolic circuit information. Furthermore, by setting a matter in vivo (e.g., a sugar, a protein, an amino acid, a DNA, an RNA, or a signal transmitter) and acquiring the metabolic circuit information, knowledge related to a disease or the like caused by a metabolic disorder resulting from the matter in vivo can be acquired.

Still another aspect of the present invention provides the program according to the above aspect, wherein each of the basic constituent elements includes information on at least one of a substrate, a metabolite, and a matter in vivo.

This specifically depicts one example of the basic constituent elements. According to the program, each of the basic constituent elements includes information on at least one of a substrate, a metabolite, and a matter in vivo. Therefore, if the user sets an intermediate metabolite or a final metabolite such as a low-molecular compound, a sugar, a protein, or an amino acid produced by microorganisms, pharmaceuticals, pharmaceutical intermediates, alcohols, amino acids, or the like can be produced with high efficiency by acquiring optimum metabolic circuit information. Furthermore, by setting a matter in vivo (e.g., a sugar, a protein, an amino acid, a DNA, an RNA, or a signal transmitter), new metabolic circuit information related to various diseases and the like can be acquired from the simulation result.

Still another aspect of the present invention provides the program according to the above aspect, wherein each of the reaction relationships includes information on a relationship of at least one of an enzyme reaction, a transcription control reaction, a translation control reaction, and a chemical reaction between the basic constituent elements.

This specifically depicts one example of the reaction relationships. According to the program, each of the reaction relationships includes information on a relationship of at least one of an enzyme reaction, a transcription control reaction, a translation control reaction, and a chemical reaction between the basic constituent elements. Therefore, in relation to the enzyme reaction, for example, information on a synthetic rate and a decomposition rate for the enzyme reaction between a substrate and a metabolite can be defined as edge information, and simulation can be carried out based on the information. In relation to the transcription control reaction, information such as a transcription rate, a transcription promoter, and a transcription inhibitor for the transcription control reaction between a DNA and an RNA can be defined as edge information, and simulation can be carried out based on the information. In relation to the translation control reaction, a translation rate, a translation promoter, and a translation inhibitor for the translation control reaction between an mRNA and a protein can be defined as edge information, and simulation can be carried out based on the information. In relation to the chemical reaction, information such as a structural change, a polymerization property, a stability, and a chemical equilibrium for the chemical reaction between metabolites can be defined as edge information, and simulation can be carried out based on the information.

Still another aspect of the present invention provides the program according to the above aspect, wherein the environmental factor information includes information on at least one of a temperature, a pH, an atmospheric pressure, the number of times of stirring, a dissolved oxygen quantity, a culture medium component, an optical condition, and a carbon dioxide concentration.

This specifically depicts one example of the environmental factor information. According to the program, the environmental factor information includes information on at least one of a temperature, a pH, an atmospheric pressure, the number of times of stirring, a dissolved oxygen quantity, a culture medium component, an optical condition, and a carbon dioxide concentration. Therefore, simulation can be carried out based on information on an upper limit and a lower limit of a temperature, a pH, an atmospheric pressure, the number of times of stirring, a dissolved oxygen quantity, culture medium components, optical conditions, a carbon dioxide concentration, or the like that is feasible for an operation in a laboratory or a factory.

Still another aspect of the present invention provides the program according to the above aspect, wherein the end condition information includes information on at least one of a culture time, a metabolic efficiency, a metabolite quantity, the number of times of simulation, a cell density, a culture cost, a pH, a dissolved oxygen quantity, and a culture medium component quantity.

This specifically depicts one example of the end condition information. According to the program, the end condition information includes information on at least one of a culture time, a metabolic efficiency, a metabolite quantity, the number of times of simulation, a cell density, a culture cost, a pH, a dissolved oxygen quantity, and a culture medium component quantity. Therefore, if the culture time and the culture cost, for example, are set as the end condition information, the user can know an optimum simulation result within user's desired ranges of the culture time and the culture cost and thus, metabolic circuit information usable for actual production can be acquired. In addition, if the metabolic efficiency and the metabolite quantity are set as the end condition information, then simulation can be carried out until the metabolic efficiency and the metabolite quantity reach a target efficiency and a target quantity, respectively, and metabolic circuit information such as culture medium components and culture time necessary to attain the target metabolic efficiency and the target metabolite quantity can be acquired. If the cell density, the pH, the dissolved oxygen quantity, and the culture medium component quantity are set as the end condition information, then simulation can be carried out until they reach a set pH, a set culture medium component quantity, and the like, respectively, and metabolic circuit information in light of a change in a culture state can be acquired. If the number of times of simulation is set as the end condition information, optimum metabolic circuit information can be acquired by executing simulation by a certain number of times. Therefore, the disadvantage in that it takes considerable long calculation time to acquire the metabolic circuit information due to a great calculation quantity can be avoided.

The program according to still another aspect of the present invention further includes: a mutation frequency information storage step of storing mutation frequency information on a frequency with which both of or one of the one node and the one edge included in the metabolic circuit information mutates to both of or one of another one of the nodes and another one of the edges, wherein at the simulation execution step, both of or one of the one node and the one edge included in the metabolic circuit information is changed using the mutation frequency information stored at the mutation frequency information storage step.

According to the program, mutation frequency information on a frequency with which both of or one of the one node and the one edge included in the metabolic circuit information mutates to both of or one of another one of the nodes and another one of the edges is stored. Both of or one of the one node and the one edge included in the metabolic circuit information is changed using the stored mutation frequency information. Therefore, the metabolic circuit information can be efficiently changed.

Still another aspect of the present invention provides the program according to the above aspect, wherein, at the simulation execution step, both of or one of the one node and the one edge included in the metabolic circuit information is changed using at least one of a genetic algorithm and simulated annealing.

This depicts one example of the simulation execution step.
According to the program, both of or one of the one node and the one edge included in the metabolic circuit information is changed using at least one of a genetic algorithm and simulated annealing. Therefore, by applying a known optimization algorithm, the user can efficiently acquire optimum new metabolic circuit information other than stationary-state metabolic circuit information.

Moreover, the present invention relates to a recording medium. The recording medium according to still another aspect of the present invention records the program according to any one of the aspects of the invention.

According to the recording medium, by allowing a computer to read and execute the program recorded on the recording medium, the program can be realized using the computer and the same advantages as those of the programs can be achieved.

BRIEF DESCRIPTION OF THE DRAWINGS

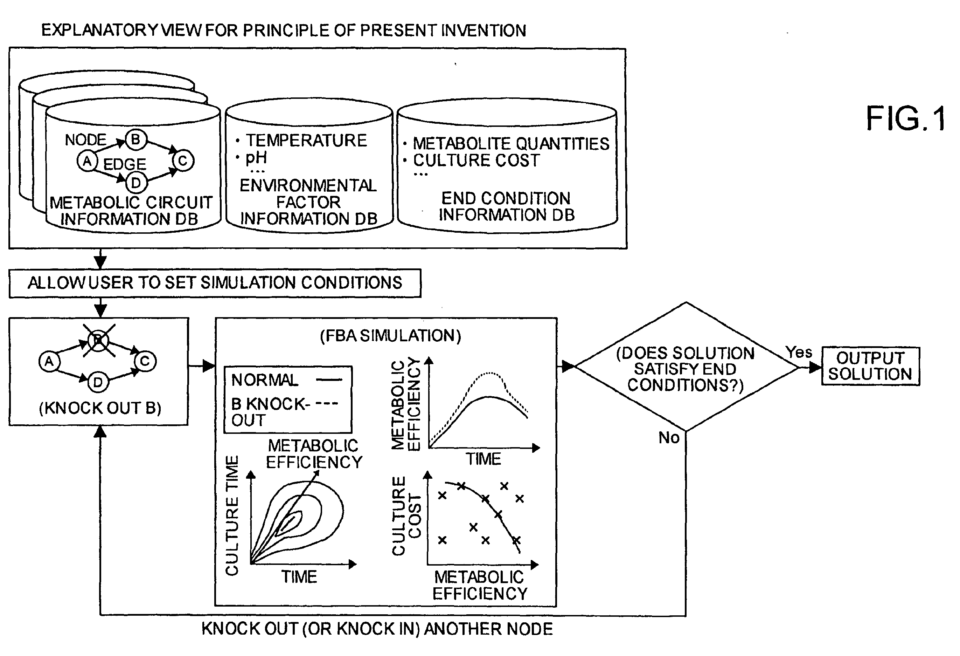

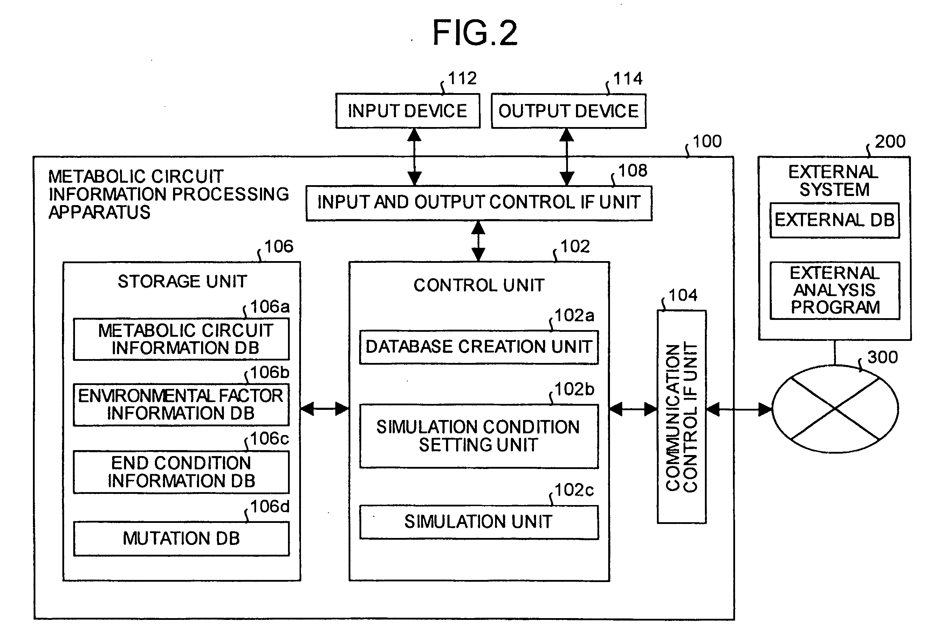

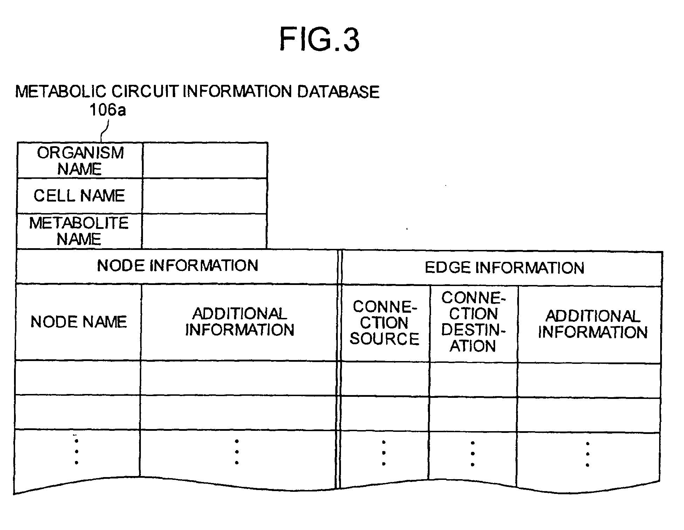

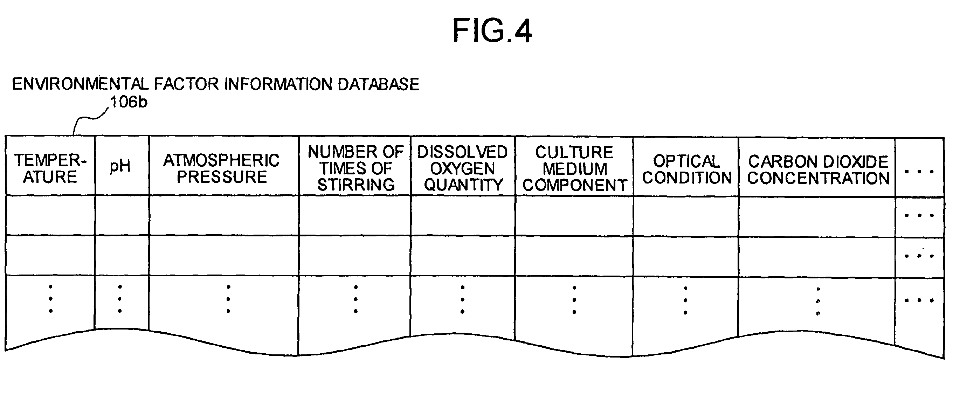

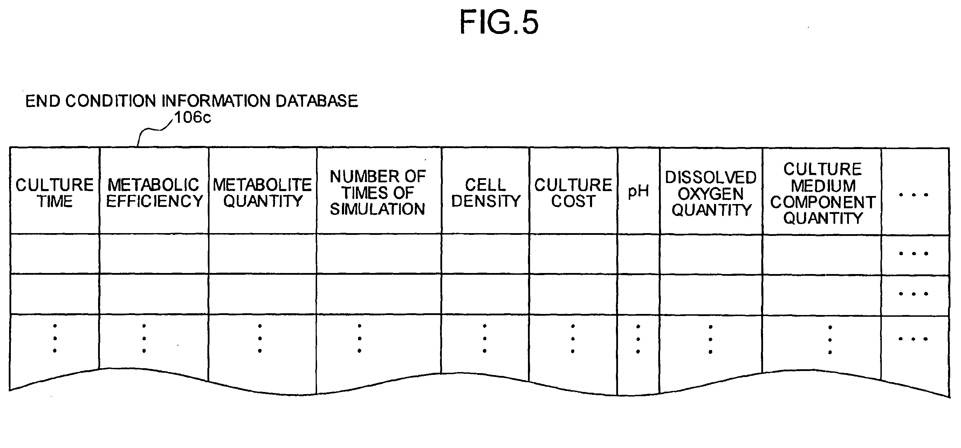

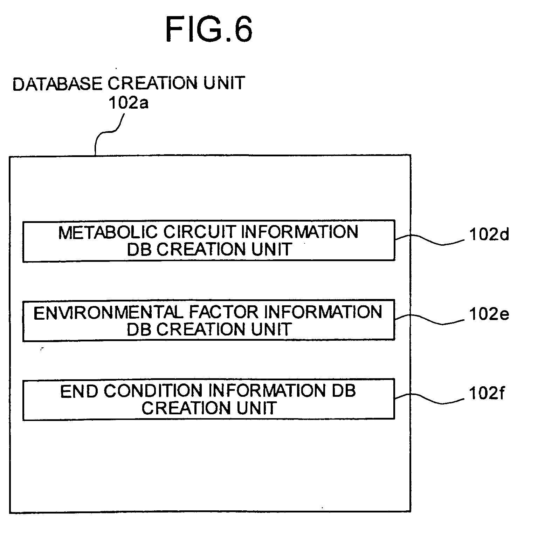

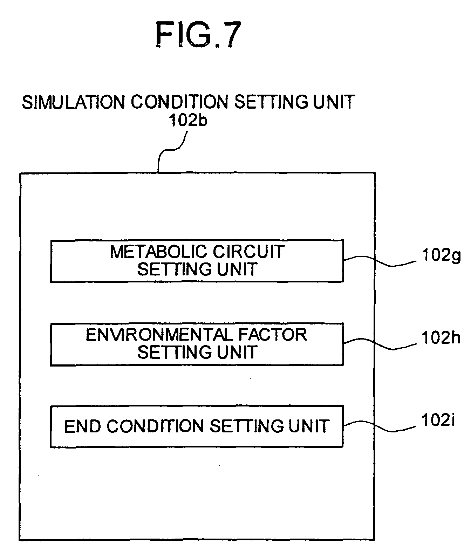

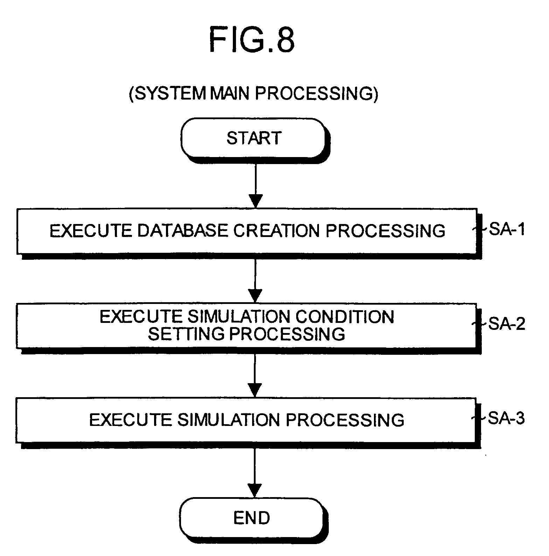

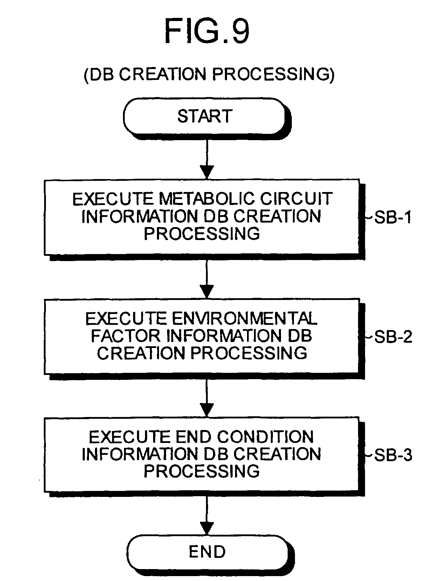

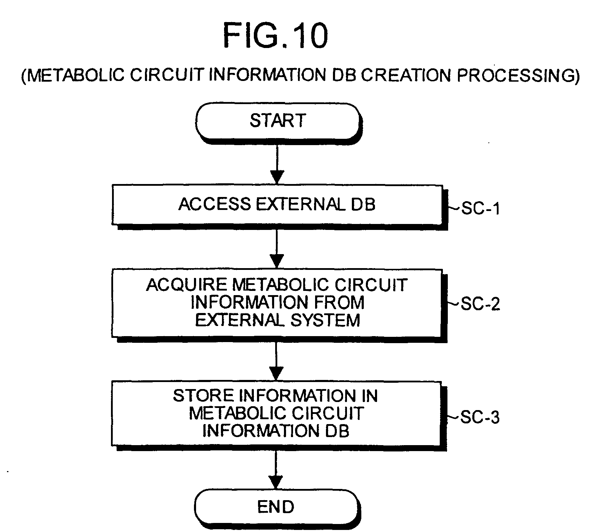

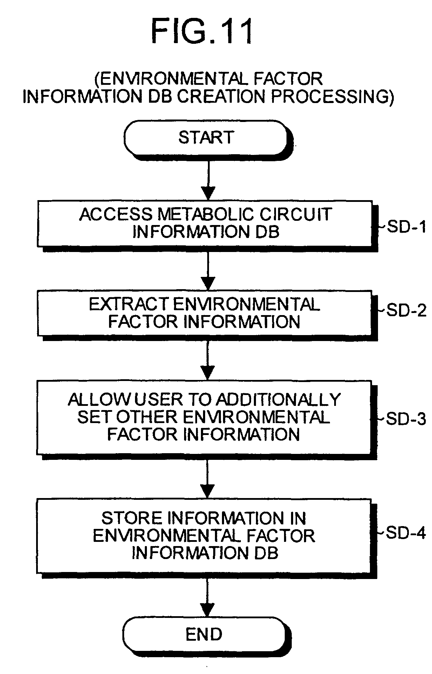

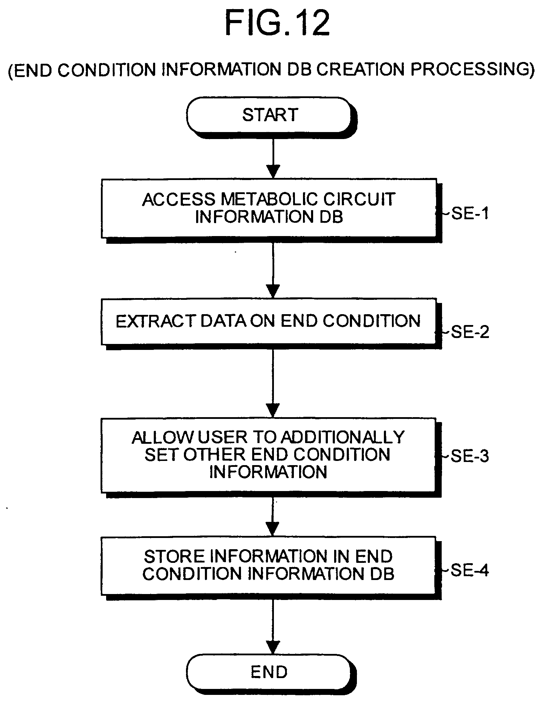

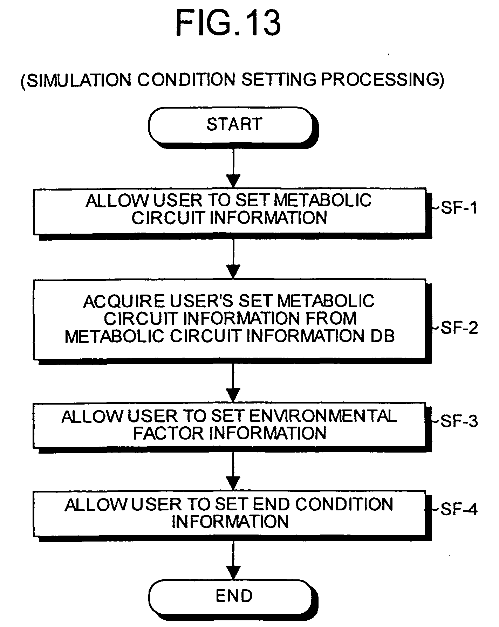

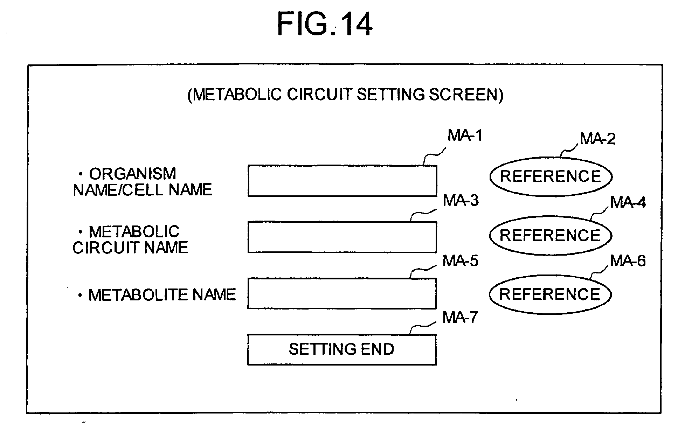

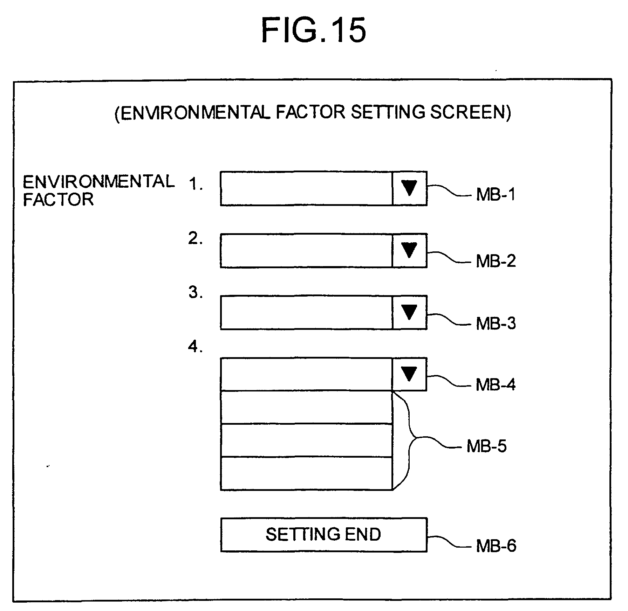

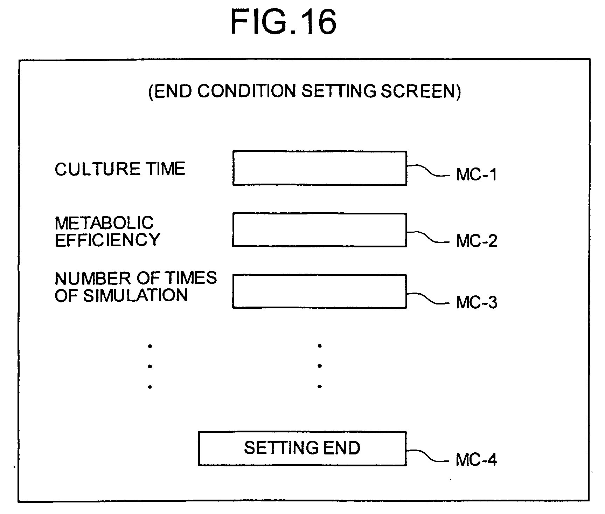

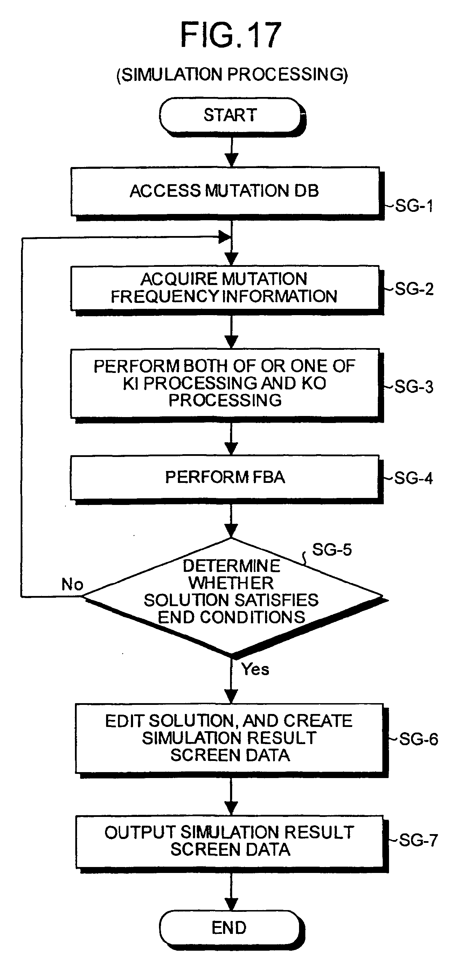

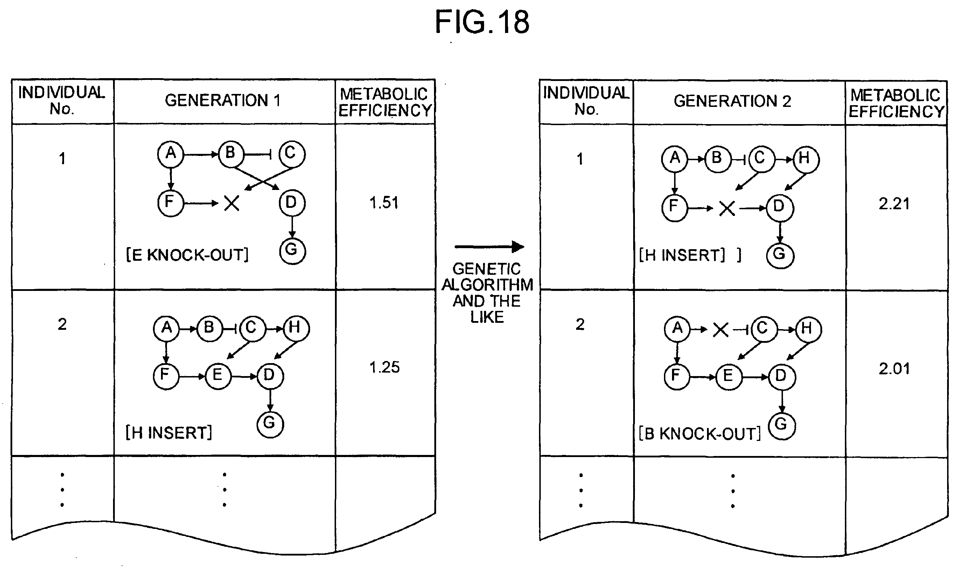

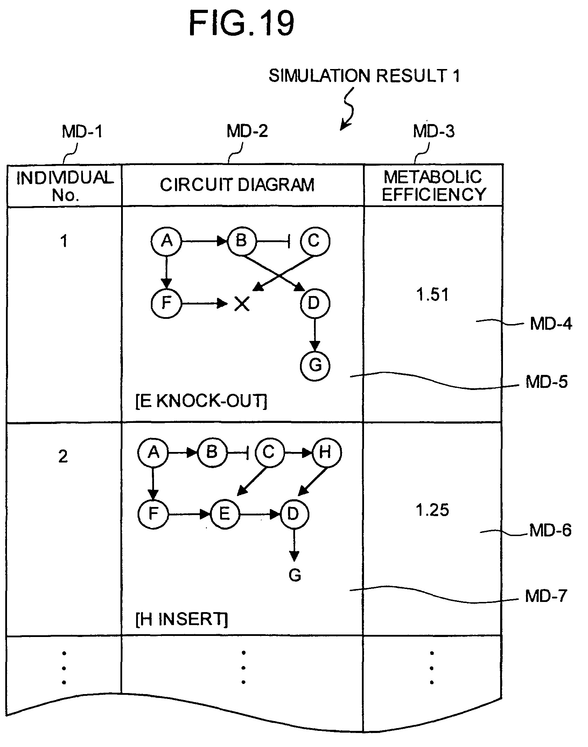

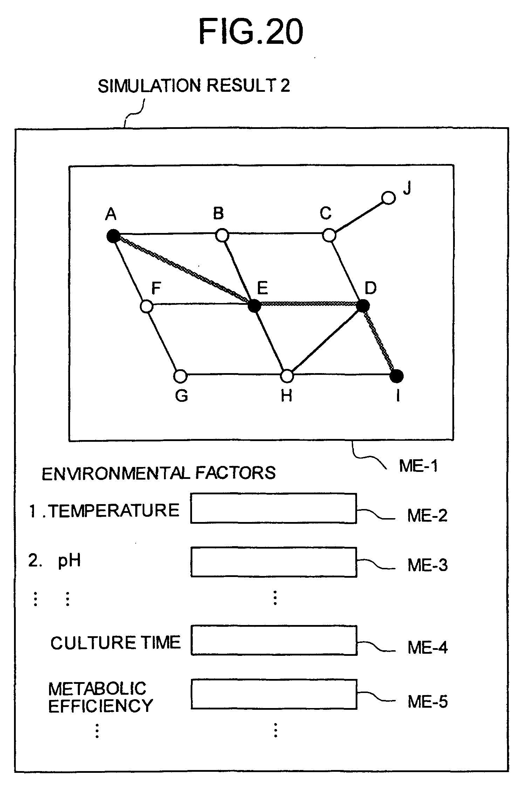

Fig. 1 is a principle explanatory view for one example of a basic principle of a system according to the present invention; Fig. 2 is a block diagram for one example of the configuration of the system to which the present invention is applied; Fig. 3 is a conceptual view for one example of information stored in a metabolic information circuit database 106a; Fig. 4 is a conceptual view for one example of information stored in an environmental factor information database 106b; Fig. 5 is a conceptual view for one example of information stored in an end condition information database 106c; Fig. 6 is a block diagram for one example of the configuration of a database creation unit 102; Fig. 7 is a block diagram for one example of the configuration of a simulation condition setting unit 102b; Fig. 8 is a flowchart for one example of a main processing performed by the system; Fig. 9 is a flowchart for one example of a database creation processing performed by the system; Fig. 10 is a flowchart for one example of a metabolic circuit information database creation processing; Fig. 11 a flowchart for one example of an environmental factor information database creation processing; Fig. 12 a flowchart for one example of an end condition information database creation processing; Fig. 13 a flowchart for one example of a simulation condition setting processing performed by the system; Fig. 14 depicts one example of a metabolic circuit setting screen output to an output device 114 by a processing performed by a metabolic circuit setting unit 102g; Fig. 15 depicts one example of an environmental factor setting screen output to the output device 114 by a processing performed by an environmental factor setting unit 102h; Fig. 16 depicts one example of an end condition setting screen output to the output device 114 by a processing performed by an end condition setting unit 102i; Fig. 17 is a flowchart for one example of a simulation processing performed by the system; Fig. 18 is a conceptual view in an instance in which a simulation is carried out by a metabolic FBA after performing a knock-in ("KI") processing or a knock-out ("KO") processing; Fig. 19 depicts one example of a simulation result display screen output to the output device 114 for a metabolic circuit information processing apparatus 100; and Fig. 20 depicts one example of the simulation result display screen output to the output device 114 for the metabolic circuit information processing apparatus 100.

BEST MODE FOR CARRYING OUT THE INVENTION

Exemplary embodiments of the metabolic circuit information processing method, the metabolic circuit information processing apparatus, the program, and the recording medium will be explained, with reference to the accompanying drawings. The present invention is not limited by the embodiments.

[Outline of System]

The principle configuration of a system according to the present invention will be explained first, followed by the configuration, processings, and the like of the system. Fig. 1 is a principle explanatory view for one example of the basic principle of the system, and conceptually depicts only units related to the present invention in the system configuration.

The system is for acquiring information on an optimum metabolic circuit for fulfilling a user's objective. A user first selects metabolic circuit information on target metabolism (e.g., metabolic circuit information on alcohol fermentation of yeasts) from a metabolic circuit information database. The metabolic circuit information database may be, for example, a database that stores metabolic circuit information acquired from external metabolic information databases such as KEGG or EcoCyc accessed through the Internet or may be a database created by storing original metabolic circuit information therein. The metabolic circuit information includes information on at least nodes and edges. The "nodes" represent herein basic constituent elements of the metabolism, for example, DNAs, RNAs, proteins, or metabolites. The "metabolites" represent herein metabolic intermediates and final metabolites. The "edges" represent herein reaction relationships among the constituent elements, for example, a relationship related to an enzyme reaction among the basic constituent elements, a relationship related to a reaction for controlling DNA transcription, a relationship related to a reaction for controlling translation from an mRNA to a protein, and a relationship related to a chemical reaction between the metabolites.

The user next selects environmental factor information related to the target yeast fermentation from an environmental factor information database, and sets a value of the selected environmental factor information. The environmental factor information database may be a database obtained by extracting environmental factor information from the metabolic information database created in advance and storing the extracted information, or may be a database obtained by user's newly adding original environmental factor information and storing the added information therein. The "environmental factor information" includes herein, for example, a culture temperature, a pH of a culture solution, an internal atmospheric pressure of a culture vessel such as a fermentor, the number of times of stirring the culture solution, a quantity of oxygen dissolved in the culture solution, quantities of culture medium components such as a carbon source, a nitrogen source, and trace metals, optical conditions such as a luminous intensity, and a carbon dioxide concentration.

The user selects end condition information on a condition for finishing the simulation from an end condition information database created in advance, and sets a value of the information. The "end condition information database" may be a database obtained by extracting information serving as end conditions from the metabolic circuit information database created in advance and storing the extracted information, or may be a database obtained by user's newly adding original end condition information and storing the added information. The "end condition information" means herein, for example, threshold information on at least one of the culture time, the metabolic efficiency, a quantity of a target metabolite, the number of times of simulations performed by the system, a cell density, a culture cost, the pH of the culture solution, the quantity of oxygen dissolved in the culture solution, and the quantities of culture medium components such as the carbon source, the nitrogen source, and the trace metals.

If the user is thus completed with settings of various conditions for a simulation processing, the metabolic circuit information processing apparatus executes the simulation processing according to these conditions. In the simulation processing, both of or one of one of the nodes and one of the edges of the metabolic circuit information selected by the user is changed, a metabolic FBA is performed until a solution satisfies the end condition information, and the solution thus obtained is stored. Changed portions of the node and the edge may be obtained by, for example, automatically selecting portions that may be highly likely changed using a mutation database that stores information on nodes and edges that tend to mutate in metabolic circuits, or by user's individually designating portions. The changed portions may he appropriately determined and optimum solution may be searched by known algorithms such as a genetic algorithm and simulated algorithm. Respective processes of the simulation processing may be performed in parallel.

The metabolic circuit information processing apparatus edits a simulation result, creates simulation result screen data, and outputs the data.

The basic concept of the present invention will be explained in more detail, taking yeast as an example. The metabolic circuit related to yeast fermentation is thoroughly studied, and principal parts of the circuit are understood though not completely. The metabolic circuit information processing apparatus specifies N pieces of environmental factor information considered to be related to the circuit. The apparatus sets a function for defining an alcohol fermentation efficiency as the end condition information. The function may be proportional to an expression quantity of a specific gene (A) or may be a function $f(X_0, X_1, ..., X_N)$ for coupling of a plurality of factors. If a metabolic FBA approach is used, a solution set of fermentation efficiencies at respective points in an N-dimensional space formed by N factors related to the fermentation and an optimum operation line maximizing the fermentation efficiencies is obtained from the given circuit. The solution obtained herein is obtained from the given circuit, i.e., the stationary-state metabolic circuit.

The metabolic circuit information processing apparatus automatically (or semiautomatically) discovers a circuit that can further improve the fermentation efficiencies when the stationary-state circuit is changed by a method such as a genetic algorithm or simulated annealing. In the genetic algorithm, for example, knock-in ("KI") and knock-out ("KO") of genes (nodes) are basically used as operation. In an all solution search, the KI and KO of all genes (nodes) are tried. This processing is repeatedly carried out until the end condition information is satisfied, and the optimum circuit at a time at which this end condition information is satisfied is set as a solution.

This process enables setting conditions so as to avoid incomprehensive mutations and experiments that require high culture cost. In addition, this process enables obtaining the optimum circuit for both the fermentation efficiencies and the culture cost.

[System Configuration]

The configuration of the system for embodying these basic features will be explained.

Fig. 2 is a block diagram for one example of the configuration of the system to which the present invention is applied. Fig. 2 conceptually depicts only units related to the present invention in the system configuration. This system is constituted so that a metabolic circuit information processing apparatus 100 that processes metabolic information, and an external system 200 that provides databases on the metabolic information and the like and external analysis programs for a metabolic information search and the like are communicably connected to each other through a network 300.

In Fig. 2, the network 300 functions to connect the metabolic circuit information processing apparatus 100 and the external system 200 to each other, and is, for example, the Internet.

In Fig. 2, the external system 200 is connected to the metabolic circuit information processing apparatus 100 through the network 300, and functions to provide the external databases on the metabolic information and the like, and websites for executing the external analysis programs for a metabolic circuit search and the like to the user.

The external system 200 may be constituted as a WEB server, an ASP server, or the like, and hardware of the external system 200 may include an information processing apparatus such as a commercially available workstation or personal computer, and accessories of the apparatus. Respective functions of the external system 200 are realized by a CPU, a disk device, a memory device, an input device, an output device, a communication control device, and the like in the hardware configuration of the external system 200 as well as programs for controlling these devices, and the like.

In Fig. 2, the metabolic circuit information processing apparatus 100 includes a control unit 102 such as the CPU for generally controlling entirety of the metabolic circuit information processing apparatus 100, a communication control interface unit 104 connected to a communication device (not shown) such as a router connected to a communication line or the like, an input and output control interface unit 108 connected to an input device 112 and an output device 114, and a storage unit 106 that stores various databases (a metabolic circuit information database 106a, an environmental factor information database 106b, an end condition information database 106c, and a mutation database 106d). The respective units are communicably connected to one another through arbitrary communication lines. In addition, this metabolic circuit information processing apparatus 100 is communicably connected to the network 300 through the communication device such as the router and a wired or wireless communication line such as a dedicated line.

The various databases stored in the storage unit 106 are storage units such as fixed disk devices, and store various programs, tables, files, databases, webpage files, and the like used for various processings.

Among the constituent elements of the storage unit 106, the metabolic circuit information database 106a is a metabolic circuit information storage unit that stores metabolic circuit information including information on the nodes that represent the basic constituent elements of the metabolism and the edges that represent reaction relationships among the basic constituent elements.

Fig. 3 is a conceptual view for one example of the information stored in the metabolic circuit information database 106a. As shown in Fig. 3, the metabolic circuit information database 106a stores index information such as organism names, cell names, and metabolism names as well as node information and edge information on every index information.

The "node information" is information stored so that the nodes such as DNAs, RNAs, proteins, and metabolites are stored while they are associated with additional information. The "additional information" means herein information such as operation conditions and physical structures of the nodes. The additional information on an enzyme, for example, includes an optimum pH, an optimum temperature, a reaction rate, a three-dimensional structure of the enzyme, a temperature range in which the enzyme is stable, and a pH range in which the enzyme is stable. The additional information on a DNA or an RNA includes a three-dimensional structure of the DNA or RNA, a name of a coded protein and a function of the protein, transcription, translation promotion, and inhibitors. The additional information on a metabolite includes a chemical structure, a molecular weight, analogues, and stability and toxicity relative to temperature and light.

The "edge information" is information on a connection source node and a connection destination node of each edge and additional information on the information that are stored while making them correspond to each other. The "additional information" on each edge includes, for example, a transcription factor type, and a DNA binding condition, a transcription rate, transcription promotion, transcription inhibitors for a transcription reaction between a node source DNA and a node destination RNA. The "additional information" on each edge also includes, for example, translation rate, translation promotion, translation inhibitors for a translation reaction between a node source mRNA and a node destination protein. The "additional information" on each edge further includes information on a structural change, a polymerization property, a stability, and a chemical equilibrium in a chemical reaction between a node source metabolic intermediate and a node destination final metabolite.

The environmental factor information database 106b is an environmental factor information storage unit that stores environmental factor information on environmental factors influencing both of or either one of the basic constituent elements and the reaction relationships. Fig. 4 is a conceptual view for one example of information stored in the environmental factor information database 106b. The "environmental factor information" includes information on at least one of the temperature, the pH, the atmospheric pressure, the number of times of stirring, the dissolved oxygen quantity, the culture medium compound, the optical condition, and the carbon dioxide concentration.

The end condition information database 106c is an end condition information storage unit that stores end condition information on conditions for finishing a simulation. Fig. 5 is a conceptual view for one example of information stored in the end condition information database 106c. The "end condition information" includes information on at least one of the culture time, the metabolic efficiency, the metabolite quantity, the number of times of simulations, the cell density, the culture cost, the pH, the dissolved oxygen quantity, the culture medium component quantities.

The mutation database 106d is a mutation frequency information storage unit that stores mutation frequency information on frequencies with which both of or one of one of the nodes and one of the edges included in the metabolic circuit information mutates to both of or one of another node and another edge. The "mutation frequency information" is information stored so that both of or one of the nodes and the edges included in the metabolic circuit information, and the mutation frequencies are defined while associating them, and are stored in a descending order of mutation frequencies.

In Fig. 2, the communication control interface unit 104 controls communication between the metabolic circuit information processing apparatus 100 and the network 300 (or communication device such as router). The communication control interface unit 104 has a function of communicating data with other terminals through a communication line.

In Fig. 2, the input and output control interface unit 108 controls the input device 112 and the output device 114. As the output device 114, a monitor (including a home television set), a loudspeaker or the like can be used (it is noted that the output device 114 is sometimes referred to as "monitor" hereafter). As the input device 112, a keyboard, a mouse, a microphone, or the like can be used. The monitor also realizes a pointing device function in cooperation with the mouse.

In Fig. 2, the control unit 102 includes an internal memory for storing various programs such as an OS (Operating System), programs for specifying various processing procedures, and required data. Using these programs and the like, information processings for executing various processings are performed. The control unit 102 functionally conceptually includes a database creation unit 102a, a simulation condition setting unit 102b, and a simulation unit 102c.

The database creation unit 102a is a database creation unit that creates various databases. The simulation condition setting unit 102b allows the user to set at least one of desired metabolic circuit information, environmental factor information, and end condition information. The simulation unit 102c changes both of or one the one node and the one edge included in the metabolic circuit information until the end condition information set by the simulation condition setting unit is satisfied, and executes a metabolic simulation by the metabolic FBA.

Fig. 6 is a block diagram for one example of the configuration of the database creation unit 102a, and conceptually depicts only units related to the present invention in the configuration. The database creation unit 102a includes a metabolic circuit information database creation unit 102d, an environmental factor database creation unit 102e, and an end condition information database creation unit 102f.

Among the constituent elements of the database creation unit 102a, the metabolic circuit information database creation unit 102d selects metabolic circuit related information such as enzyme information, gene control information, signal transmission information related to various metabolic circuits and metabolisms from the external metabolic information databases, and stores the selected information in the metabolic circuit information database 106a.

The environmental factor information database creation unit 102e selects environmental factor related information from the metabolic circuit information database 106a, and stores the selected information in the environmental factor information database 106b. The environmental factor information database creation unit 102e allows the user to add new environmental factor information and stores the added information.

The end condition information database creation unit 102f selects end condition related information from the metabolic circuit information database 106a and stores the selected information in the end condition information database 106c. The end condition information database creation unit 102f allows the user to newly add original end condition information and stores the added information. Details of processings performed by the respective units will be explained later.

Fig. 7 is a block diagram for one example of the configuration of the simulation condition setting unit 102b, and conceptually depicts only units related to the present invention in the configuration. The simulation condition setting unit 102b includes a metabolic circuit setting unit 102g, an environmental factor setting unit 102h, and an end condition setting unit 102i.

Among the constituent elements of the simulation condition setting unit 102b, the metabolic circuit setting unit 102g allows the user to set target metabolic circuit information. The environmental factor setting unit 102h allows the user to set the environmental factor information. The end condition setting unit 102i allows the user to set the end condition information on the simulation. Details of processings performed by the respective units will be explained later.

[System Processings]

One example of processings performed by the system according to the embodiment configured as explained above will next be explained with reference to Figs. 8 to 20.

[System Main Processing]

The detail of a main processing performed by the system will be explained with reference to the flowchart shown in Fig. 8.

The metabolic circuit information processing apparatus 100 first executes a database creation processing to be explained later with reference to Fig. 9 by a processing performed by the database creation unit 102a (at step SA-1).

The metabolic circuit information processing apparatus 100 then executes a simulation condition setting processing to be explained later with reference to Fig. 13 by a processing performed by the simulation condition setting unit 102b (at step SA-2).

The metabolic circuit information processing apparatus 100 executes a simulation processing to be explained later with reference to Fig. 17 by a processing performed by the simulation unit 102c (at step SA-3). The main processing of the system is thus finished.

[Database Creation Processing]

The detail of the database creation processing will be explained with reference to flowcharts shown in Figs. 9 to 12. Fig. 9 is a flowchart for one example of the database creation processing performed by the system according to this embodiment.

The metabolic circuit information processing apparatus 100 first creates the metabolic circuit information database by a processing performed by the metabolic circuit information database creation unit 102d (at step SB-1).

Fig. 10 is a flowchart for one example of a metabolic circuit information database creation processing. The metabolic circuit information database creation unit 102d accesses the external databases through the communication control interface unit 104 (at step SC-1), and acquires metabolic circuit information designated by the user through the input device 112 (at step SC-2). The metabolic circuit information database creation unit 102d stores the acquired metabolic circuit information in a predetermined storage area of the metabolic circuit information database 106a (at step SC-3).

Referring back to Fig. 9, the metabolic circuit information processing apparatus 100 creates the environmental factor information database by a processing performed by the environmental factor information database creation unit 102e (at step SB-2).

Fig. 11 is a flowchart for one example of an environmental factor information database creation processing. The environmental factor information database creation unit 102e accesses the metabolic circuit information database (at step SD-1), and extracts information serving as environmental factor information from the additional information on node information, that on edge information, and the like (at step SD-2). The environmental factor information database creation unit 102e displays the extracted environmental factor information on the output device 114 and allows the user to input other environmental factor information (at step SD-3), and stores the input information in the environmental factor information database 106b (at step SD-4).

Referring back to Fig. 9, the metabolic circuit information processing apparatus 100 creates the end condition information database by a processing performed by the end condition information database creation unit 102f (at step SB-3).

Fig. 12 is a flowchart for one example of an end condition information database creation processing. The end condition information database creation unit 102f accesses the metabolic circuit information database (at step SE-1), and extracts information serving as end conditions from the additional information on the node information, that on the edge information, and the like (at step SE-2). The end condition information database creation unit 102f displays the extracted end condition information on the output device 114 and allows the user to input other end condition information (at step SE-3), and stores the input information in the end condition information database 106c (at step SE-4). The database creation processing is thus finished.

[Simulation Condition Setting Processing]

The detail of the simulation condition setting processing will be explained with reference to Fig. 13. Fig. 13 is a flowchart for one example of the simulation condition setting processing performed by the system according to this embodiment.

The metabolic circuit information processing apparatus 100 allows the user to set target metabolic circuit information from the metabolic circuit information stored in the metabolic circuit information database 106a by a processing performed by the metabolic circuit setting unit 102g (at step SF-1).

Fig. 14 depicts one example of a metabolic circuit setting screen output to the output device 114 by the processing performed by the metabolic circuit setting unit 102g. As shown in Fig. 14, the metabolic circuit setting screen includes an organism name and cell name input area MA-1, a reference button MA-2 for referring to cell information stored in the metabolic circuit information database 106a, a metabolic circuit name input area MA-3, a reference button MA-4 for referring to the metabolic circuit information stored in the metabolic circuit information database 106a, a metabolite name input area MA-5, a reference button MA-6 for referring to metabolite information stored in the metabolic circuit information database 106a, a setting end button MA-7, and the like.

If the user selects the reference buttons MA-2, MA-4, and MA-6 shown in Fig. 14 using the input device 112 while viewing the metabolic circuit setting screen, the metabolic circuit setting unit 102g displays the metabolic circuit information stored in the metabolic circuit information database 106a on a display.

Thereafter, if the user completes inputting respective items of the input areas MA-1, MA-3, and MA-5 using the input device 112 and then selects the setting end button MA-7 while viewing the metabolic circuit setting screen, the metabolic circuit setting unit 102g stores the input information in the storage unit 106.

The metabolic circuit setting unit 102g accesses the metabolic circuit information database 106a, and acquires the metabolic circuit information set by the user (at step SF-2).

The metabolic circuit information processing apparatus 100 allows the user to set desired environmental factor information from the environmental factor information stored in the environmental factor information database 106b by a processing performed by the environmental factor setting unit 102h (at step SF-3). Namely, the environmental factor setting unit 102h allows the user to set various conditions for the simulation processing.

Fig. 15 depicts one example of an environmental factor setting screen output to the output device 114 by the processing performed by the environmental factor setting unit 102h. As shown in Fig. 15, environmental factor setting screen includes environmental factor input areas MB-1 to MB-4, a pull-down menu display area MB-5 for selecting information stored in the environmental factor information database 106b, a setting end button MA-6, and the like. The pull-down menu display area MB-5 displays, when the user indicates an arrow portion of one of the input areas MB-1 to MB-4 using the input device 112 such as the mouse, selectable environmental factors below the indicated input area while being superposed one after another.

If the user completes inputting respective items of the input areas MB-1 to MB-4 using the input device 112 and then selects the setting end button MB-6 while viewing the environmental factor setting screen shown in Fig. 15, the environmental factor setting unit 102h stores the input information in the storage unit 106.

The metabolic circuit information processing apparatus 100 allows the user to set desired end condition information from the end condition information stored in the end condition information database 106c by a processing performed by the end condition setting unit 102i (at step SF-4). The end condition setting unit 102i allows the user to set various conditions for finishing the simulation.

Fig. 16 depicts one example of an end condition setting screen output to the output device 114 by the processing performed by the end condition setting unit 102i. As shown in Fig. 16, the end condition setting screen includes a culture time input area MC-1, a metabolic efficiency input area MC-2, a number-of-times-of-simulation input area MC-3, a setting end button MC-4, and the like.

If the user completes inputting respective items of the input areas MC-1 to MC-3 using the input device 112 and then selects the setting end button MC-4 while viewing the end condition setting screen shown in Fig. 16, the end condition setting unit 102i stores the input information in the storage unit 106. The simulation condition setting processing is thus finished.

[Simulation Processing]

The detail of the simulation processing will be explained with reference to Fig. 17. Fig. 17 is a flowchart for one example of the simulation processing performed by the system according to this embodiment.

The simulation unit 102c first accesses the mutation database 106d (at step SG-1), and acquires the mutation frequency information on frequencies with which both of or one of the one node and the one edge included in the metabolic circuit information mutates to both of or one of another node and another edge (at step SG-2).

The simulation unit 102c performs both of or one of a knock-in (KI) processing and a knock-out (KO) processing for the nodes and edges (at step SG-3) based on the mutation frequency information.

"Both of or one of a knock-in (KI) processing and a knock-out (KO) processing" means to conduct both of or one of insertion (KI) and reduction (KO) for the nodes and the edges in the metabolic circuit information based on the mutation frequency information and the tike. For example, the simulation unit 102c conducts both of or one of the insertion (KI) and the reduction (KO) for the nodes and the edges in the descending order of mutation frequencies based on the mutation frequency information and the like.

The simulation unit 102c performs a metabolic FBA for the metabolic circuit information changed by both of or one of the knock-in (KI) processing and the knock-out (KO) processing (at step SG-4). Namely, the simulation unit 102c changes both of or one of the one node and the one edge included in the target metabolic circuit information, and executes the metabolic simulation by the metabolic FBA using data on the environmental factor information thus set.

Fig. 18 is a conceptual view for when the simulation unit 102c performs the simulation by the metabolic FBA after performing both of or one of the knock-in (KI) processing and the knock-out (KO) processing. As shown in Fig. 18, the simulation unit 102c performs the simulation by the metabolic FBA based on the metabolic circuit information for which both of or one of the KI and KO processings is executed and the environmental factor information, and calculates the metabolic efficiency or the like of the metabolic circuit after both of or one of the KI and KO.

For example, if a concentration of a metabolic product i, a metabolite synthetic rate, a decomposition rate, a consumption rate of metabolic reactants necessary for growth and the like, a rate of a concentration change caused by the transfer of metabolites in system boundaries defined for the analysis are set as the additional information on the edges, and if the metabolic circuit obtained by conducting both of or one of the KI processing and the KO processing to the nodes and edges related to the additional information is represented by Equation 1;

$$dX_i/dt = V_{syn} - V_{deg} - V_{use} \pm V_{trans} \text{ (Equation 1)} \qquad \text{(Equation 1)}$$

(wherein, $X_i$ is the concentration of the metabolic product i, $V_{syn}$ is the synthetic rate of the metabolite i, $V_{deg}$ is the decomposition rate, $V_{use}$ is the consumption rate of the metabolic reactants necessary for growth and the like, and $V_{trans}$ is the rate of the concentration change caused by the transfer of the metabolites in the system boundaries defined for analysis), the Equation 1 is further represented by a linear algebra. Thereafter, a basis of a resultant matrix is transformed into a biochemically significant basis. Vectors of this basis correspond to specific metabolic pathways, respectively. Based on this basis, it is examined whether the metabolic circuit is optimized for some function. For example, two variables related to the objective function (metabolic efficiency or the like) are picked up, and an optimum metabolic balance is calculated for all points on planes of the two variables using linear programming. If highest metabolic efficiency is set as the end conditions, for example, a function having the highest metabolic efficiency is selected. As a selection method, a known optimization algorithm such as the genetic algorithm or the simulated annealing may be used.

The simulation unit 102c determines whether a simulation result (solution) satisfies the end condition information set by the simulation condition setting unit (at step SG-5). If the simulation result (solution) does not satisfy the end condition information, the processing returns to the step SG-2. Namely, the simulation unit 102c determines whether the simulation result satisfies data on the end condition information thus set. If determining that the simulation result does not satisfy the data, the simulation unit 102c changes both of or one of another node and another edge and executes a simulation.

If determining that the simulation result satisfies the end condition information, the simulation unit 102c creates the simulation result screen data (at step SG-6), and outputs the created data to the output device 114 (at step SG-7).

Each of Figs. 19 and 20 depicts one example of a simulation result display screen output to the output device 114 for the metabolic circuit information processing apparatus 100. As shown in Figs. 19 and 20, the simulation unit 102c outputs a changed metabolic circuit diagram, a changed numeric value, a metabolic efficiency, and the like as the simulation results, in the form of graphs.

In the example of the simulation result display screen shown in Fig. 19 (simulation result 1), a header includes an individual number MD-1, a changed metabolic circuit diagram MD-2 changed by this simulation processing, and a metabolic efficiency MD-3 calculated by the metabolic FBA based on the changed metabolic circuit diagram. As shown in Fig. 19, the metabolic circuit diagrams may be displayed while being sorted in an order of metabolic efficiencies. In Fig. 19, the circuit diagram corresponding to an individual number 1 is MD-5, and the metabolic efficiency of the metabolic circuit is MD-4. The circuit diagram corresponding to an individual number 2 is MD-7, and the metabolic efficiency of the metabolic circuit is MD-6.

In the example of the simulation result display screen shown in Fig. 20 (simulation result 2), the simulation unit 102c includes a display area ME-1 for the metabolic circuit diagram that produces an optimum simulation result, and display areas ME-2 to ME-5 for numerical values of various pieces of environmental factor information when the optimum simulation result is produced. As shown in Fig. 20, both a stationary-state (default) metabolic circuit and a changed metabolic circuit may be discriminately displayed in the metabolic circuit diagram display area ME-1, by indicating edges of the stationary-state (default) metabolic circuit by thin lines and nodes thereof by white circles or the like, and by indicating edges of the changed metabolic circuit by bold lines and nodes thereof by black circles or the like. The simulation processing is thus finished.

[Other Embodiments]

Exemplary embodiments of the present invention are described above, however, variously modified embodiments other than the one described can be made within the scope of the technical spirit of the appended claims.

Further, among the respective processings explained in the embodiments, all of or part of the processings explained to be performed automatically may be performed manually or all of or part of the processings explained to be performed manually may be performed automatically by a well-known method.

The processing procedures, control procedures, specific names, information including various pieces of registered data, parameters for search conditions, and the like, screen examples, and database configurations explained above or shown in the drawings may be arbitrarily changed unless specified otherwise.

The respective constituent elements of the metabolic circuit information processing apparatus 100 shown in the drawings are functionally conceptual, and the metabolic circuit information processing apparatus 100 is not always required to be physically configured as shown in the drawings.

For example, all of or arbitrary part of the processing functions of the respective servers provided in the metabolic circuit information processing apparatus 100, particularly the respective processing functions performed by the control unit 102 can be realized by the CPU (Central Processing Unit) and programs interpreted and executed by the CPU, or can be realized as hardware based on wired logic. The programs are recorded on the recording medium to be explained later, and mechanically read by the metabolic circuit information processing apparatus 100 as needed.

The various databases and the like (the metabolic circuit information database 106a, the environmental factor information database 106b, the end condition information database 106c, and the mutation database 106d) stored in the storage unit 106 are storage units such as memory devices, for example, a RAM and a ROM, fixed disk devices, for example, a hard disk, a flexible disk, and an optical disk. They store various programs, tables, files, databases, webpage files, and the like used for various processings and provision of websites.

In addition, the metabolic circuit information processing apparatus 100 may be realized by connecting peripherals such as a printer, a monitor, and an image scanner to an information processing apparatus such as an information processing terminal, for example, a well-known personal computer or workstation, and by installing software (including a program, data, or the like) for realizing the method of the present invention into the information processing apparatus.

The specific form of distribution and integration of the metabolic circuit information processing apparatus 100 is not limited to that shown in the drawings. All of or part of the metabolic circuit information processing apparatus 100 can be functionally or physically distributed or integrated in arbitrary units according to various loads and the like. For example, each database may be constituted independently as an independent database device, and part of the processings may be realized using a CGI (Common Gateway Interface).

Further, the program according to the present invention can be stored in a computer readable recording medium. It is assumed herein that examples of this "recording medium" include arbitrary "portable physical mediums" such as a floppy disk (registered trademark), a magneto-optical disk, a ROM, an EPROM, an EEPROM, a CD-ROM, an MO, and a DVD, arbitrary "fixed physical mediums" such as a ROM, a RAM, and an HD included in various computer systems, and "communication mediums" that temporarily hold the program such as a communication line or a carrier wave used when the program is transmitted through the network represented by a LAN, a WAN, or the Internet.

The "program" is a data processing method described in an arbitrary language or by an arbitrary description method, and the form of the "program" is not limited but may be a source code, a binary code, or the like. The "program" is not limited to a program constituted as a single program. Examples of the "program" include a program constituted to be distributed as a plurality of modules or libraries, and a program that fulfils its function in cooperation with another program represented by the OS (Operating System). The specific configurations, reading procedures, install procedures after reading, and the like of the respective devices shown in the embodiment for reading the recording medium may be well-known configurations and procedures.

Furthermore, the network 300 functions to connect the metabolic circuit information processing apparatus 100 and the external system 200 to each other, and may include any one of, for example, the Internet, an Intranet, a LAN (which may be either wired or wireless), a VAN, a personal computer communication network, a public telephone network (which may be either analog or digital), a dedicated line network (which may be either analog or digital), a CATV network, a portable line exchange network/portable packet exchange network such as an IMT 2000 network, a GSM network, or a PDC/PDC-P network, a wireless call network, a local wireless network such as Bluetooth, a PHS network, and satellite communications network such as CD, BS, or ISDB. That is, the present system can transmit and receive various pieces of data through an arbitrary network whether the system is wired or wireless.

As explained so far in detail, the present invention allows a user to set target metabolic circuit information that includes information on nodes respectively representing basic constituent elements of a metabolism and edges respectively representing reaction relationships among the basic constituent elements; allows the user to set environmental factor information on an environmental factor that influences both of or one of the basic constituent elements and the reaction relationships; allows the user to set end condition information on a condition for finishing a simulation;

changes both of or one of one of the nodes and one of the edges included in the target metabolic circuit information, and executes a metabolic simulation by a metabolic FBA using the set environmental factor information; determines whether a result of the simulation satisfies the set end condition information, and, if the result of the simulation does not satisfy the end condition information, changes both of or one of another one of the nodes and another one of the edges, followed by execution of the simulation execution step; and outputs the result of the executed simulation. Therefore, the present invention can provide a metabolic circuit information processing method, a metabolic circuit information processing apparatus, a program, and a recording medium that can realize the following respects. The user can easily set various conditions according to a user's objective, and a user's target optimum metabolic circuit information or new metabolic circuit information can be acquired by performing a simulation processing. By user's setting, for example, a toxic matter such as dioxin or a persistent matter such as plastic as a substrate (starting material) and obtaining information on an optimum metabolic circuit for metabolizing the substrate, the information can be used for biodegradation of toxic matters and persistent matters. In addition, if the user sets an intermediate metabolite or a final metabolite such as a low-molecular compound, a sugar, a protein, or an amino acid, then optimum metabolic circuit information for producing metabolites can be acquired from a simulation result, and pharmaceuticals and foods can be produced using this metabolic circuit information. Furthermore, by setting a matter in vivo (e.g., a sugar, a protein, an amino acid, a DNA, an RNA, or a signal transmitter) and acquiring the metabolic circuit information, knowledge related to a disease or the like caused by a metabolic disorder resulting from the matter in vivo can be acquired.

According to the present invention, each of the basic constituent elements includes information on at least one of a substrate, a metabolite, and a matter in vivo. Therefore, the present invention can provide a metabolic circuit information processing method, a metabolic circuit information processing apparatus, a program, and a recording medium that can realize the following respects. If the user sets an intermediate metabolite or a final metabolite such as a low-molecular compound, a sugar, a protein, or an amino acid produced by microorganisms, pharmaceuticals, pharmaceutical intermediates, alcohols, amino acids, or the like can be produced with high efficiency by acquiring optimum metabolic circuit information. Furthermore, by setting a matter in vivo (e.g., a sugar, a protein, an amino acid, a DNA, an RNA, or a signal transmitter), new metabolic circuit information related to various diseases and the like can be acquired.

According to the present invention, each of the reaction relationships includes information on a relationship of at least one of an enzyme reaction, a transcription control reaction, a translation control reaction, and a chemical reaction between the basic constituent elements. Therefore, the present invention can provide a metabolic circuit information processing method, a metabolic circuit information processing apparatus, a program, and a recording medium that can realize the following respects. In relation to the enzyme reaction, for example, information on a synthetic rate and a decomposition rate for the enzyme reaction between a substrate and a metabolite can be defined as edge information, and simulation can be carried out based on the information. In relation to the transcription control reaction, information such as a transcription rate, a transcription promoter, and a transcription inhibitor for the transcription control reaction between a DNA and an RNA can be defined as edge information, and simulation can be carried out based on the information. In relation to the translation control reaction, a translation rate, a translation promoter, and a translation inhibitor for the translation control reaction between an mRNA and a protein can be defined as edge information, and simulation can be carried out based on the information. In relation to the chemical reaction, information such as a structural change, a polymerization property, a stability, and a chemical equilibrium for the chemical reaction between metabolites can be defined as edge information, and simulation can be carried out based on the information.

According to the present invention, the environmental factor information includes information on at least one of a temperature, a pH, an atmospheric pressure, the number of times of stirring, a dissolved oxygen quantity, a culture medium component, an optical condition, and a carbon dioxide concentration. Therefore, the present invention can provide a metabolic circuit information processing method, a metabolic circuit information processing apparatus, a program, and a recording medium that can realize the following respects. Simulation can be carried out based on information on an upper limit and a lower limit of a temperature, a pH, an atmospheric pressure, the number of times of stirring, a dissolved oxygen quantity, culture medium components, optical conditions, a carbon dioxide concentration, or the like that is feasible for an operation in a laboratory or a factory.

According to the present invention, the end condition information includes information on at least one of a culture time, a metabolic efficiency, a metabolite quantity, the number of times of simulation, a cell density, a culture cost, a pH, a dissolved oxygen quantity, and a culture medium component quantity. Therefore, the present invention can provide a metabolic circuit information processing method, a metabolic circuit information processing apparatus, a program, and a recording medium that can realize the following respects. If the culture time and the culture cost, for example, are set as the end condition information, the user can know an optimum simulation result within user's desired ranges of the culture time and the culture cost and thus, metabolic circuit information usable for actual production can be acquired. In addition, if the metabolic efficiency and the metabolite quantity are set as the end condition information, then simulation can be carried out until the metabolic efficiency and the metabolite quantity reach a target efficiency and a target quantity, respectively, and metabolic circuit information such as culture medium components and culture time necessary to attain the target metabolic efficiency and the target metabolite quantity can be acquired. If the cell density, the pH, the dissolved oxygen quantity, and the culture medium component quantity are set as the end condition information, then simulation can be carried out until they reach a set pH, a set culture medium component quantity, and the like, respectively, and metabolic circuit information in light of a change in a culture state can be acquired. If the number of times of simulation is set as the end condition information, optimum metabolic circuit information can be acquired by executing simulation by a certain number of times. Therefore, the disadvantage in that it takes considerable long calculation time to acquire the metabolic circuit information due to a great calculation quantity can be avoided.

According to the present invention, mutation frequency information on a frequency with which both of or one of the one node and the one edge included in the metabolic circuit information mutates to both of or one of another one of the nodes and another one of the edges is stored. Both of or one of the one node and the one edge included in the metabolic circuit information is changed using the stored mutation frequency information. Therefore, the present invention can provide a metabolic circuit information processing method, a metabolic circuit information processing apparatus, a program, and a recording medium that can efficiently change metabolic circuit information.

According to the present invention, both of or one of the one node and the one edge included in the metabolic circuit information is changed using at least one of a genetic algorithm and simulated annealing. Therefore, the present invention can provide a metabolic circuit information processing method, a metabolic circuit information processing apparatus, a program, and a recording medium capable of efficiently acquiring optimum new metabolic circuit information other than stationary-state metabolic circuit information by applying a known optimization algorithm.

INDUSTRIAL APPLICABILITY

As explained so far, according to the metabolic circuit information processing method, the metabolic circuit information processing apparatus, the program, and the recording medium according to the present invention, by user's setting, for example, a toxic matter such as dioxin or a persistent matter such as plastic as a substrate (starting material) and obtaining information on an optimum metabolic circuit for metabolizing the substrate, the information can be used for biodegradation of toxic matters and persistent matters. In addition, if the user sets an intermediate metabolite or a final metabolite such as a low-molecular compound, a sugar, a protein, or an amino acid, then optimum metabolic circuit information for producing metabolites can be acquired from a simulation result, and pharmaceuticals and foods can be produced using this metabolic circuit information. Furthermore, by setting a matter in vivo (e.g., a sugar, a protein, an amino acid, a DNA, an RNA, or a signal transmitter) and acquiring the metabolic circuit information, knowledge related to a disease or the like caused by a metabolic disorder resulting from the matter in vivo can be acquired.

**Claims**

1.  A metabolic circuit information processing method comprising:

    a metabolic circuit information setting step of allowing a user to set target metabolic circuit information that includes information on nodes respectively representing basic constituent elements of a metabolism and edges respectively representing reaction relationships among the basic constituent elements;
    an environmental factor information setting step of allowing the user to set environmental factor information on an environmental factor that influences both of or one of the basic constituent elements and the reaction relationships;
    an end condition information setting step of allowing the user to set end condition information on a condition for finishing a simulation;
    a simulation execution step of changing both of or one of one of the nodes and one of the edges included in the target metabolic circuit information, and of executing a metabolic simulation by a metabolic flux balance analysis using the environmental factor information set at the environmental factor information setting step;
    a simulation result determination step of determining whether a result of the simulation executed at the simulation execution step satisfies the end condition information set at the end condition information setting step, and of, if the result of the simulation does not satisfy the end condition information, changing both of or one of another one of the nodes and another one of the edges, followed by execution of the simulation execution step; and
    a simulation result output step of outputting the result of the simulation executed at the simulation execution step.

2.  The metabolic circuit information processing method according to claim 1, wherein each of the basic constituent elements includes information on at least one of a substrate, a metabolite, and a matter in vivo.

3. The metabolic circuit information processing method according to claim 1, wherein each of the reaction relationships includes information on a relationship of at least one of an enzyme reaction, a transcription control reaction, a translation control reaction, and a chemical reaction between the basic constituent elements.

4. The metabolic circuit information processing method according to claim 1, wherein the environmental factor information includes information on at least one of a temperature, a pH, an atmospheric pressure, the number of times of stirring, a dissolved oxygen quantity, a culture medium component, an optical condition, and a carbon dioxide concentration.

5. The metabolic circuit information processing method according to claim 1, wherein the end condition information includes information on at least one of a culture time, a metabolic efficiency, a metabolite quantity, the number of times of simulation, a cell density, a culture cost, a pH, a dissolved oxygen quantity, and a culture medium component quantity.

6. The metabolic circuit information processing method according to claim 1, further comprising:

   a mutation frequency information storage step of storing mutation frequency information on a frequency with which both of or one of the one node and the one edge included in the metabolic circuit information mutates to both of or one of another one of the nodes and another one of the edges, wherein
   at the simulation execution step, both of or one of the one node and the one edge included in the metabolic circuit information is changed using the mutation frequency information stored at the mutation frequency information storage step.

7. The metabolic circuit information processing method according to claim 1, wherein at the simulation execution step, both of or one of the one node and the one edge included in the metabolic circuit information is changed using at least one of a genetic algorithm and simulated annealing.

8. A metabolic circuit information processing apparatus comprising:

   a metabolic circuit information storage unit that stores metabolic circuit information including information on nodes respectively representing basic constituent elements of a metabolism and edges respectively representing reaction relationships among the basic constituent elements;
   an environmental factor information storage unit that stores environmental factor information on an environmental factor that influences both of or one of the basic constituent elements and the reaction relationships;
   an end condition information storage unit that stores end condition information on condition for finishing a simulation;
   a simulation condition setting unit that allows a user to set at least one of the metabolic circuit information, the environmental factor information, and the end condition information desired by the user;
   a simulation execution unit that changes both of or one of one of the nodes and one of the edges included in the metabolic circuit information, and executes a metabolic simulation by a metabolic flux balance analysis based on at least one of the metabolic circuit information set by simulation condition setting unit and the environmental factor information until the end condition information set by the simulation condition setting unit is satisfied; and
   a simulation result output unit that outputs a result of the simulation executed by the simulation execution unit.

9. The metabolic circuit information processing apparatus according to claim 8, wherein each of the basic constituent elements includes information on at least one of a substrate, a metabolite, and a matter in vivo.

10. The metabolic circuit information processing apparatus according to claim 8, wherein each of the reaction relationships includes information on a relationship of at least one of an enzyme reaction, a transcription control reaction, a translation control reaction, and a chemical reaction between the basic constituent elements.

11. The metabolic circuit information processing apparatus according to claim 8, wherein the environmental factor information includes information on at least one of a temperature, a pH, an atmospheric pressure, the number of times of stirring, a dissolved oxygen quantity, a culture medium component, an optical condition, and a carbon dioxide concentration.

**12.** The metabolic circuit information processing apparatus according to claim 8, wherein the end condition information includes information on at least one of a culture time, a metabolic efficiency, a metabolite quantity, the number of times of simulation, a cell density, a culture cost, a pH, a dissolved oxygen quantity, and a culture medium component quantity.

**13.** The metabolic circuit information processing apparatus according to claim 8, further comprising:

a mutation frequency information storage unit of storing mutation frequency information on a frequency with which both of or one of the one node and the one edge included in the metabolic circuit information mutates to both of or one of another one of the nodes and another one of the edges, wherein
at the simulation execution unit, both of or one of the one node and the one edge included in the metabolic circuit information is changed using the mutation frequency information stored at the mutation frequency information storage unit.

**14.** The metabolic circuit information processing apparatus according to claim 8, wherein at the simulation execution unit, both of or one of the one node and the one edge included in the metabolic circuit information is changed using at least one of a genetic algorithm and simulated annealing.

**15.** A program for allowing a computer to execute a metabolic circuit information processing method, comprising:

a metabolic circuit information setting step of allowing a user to set target metabolic circuit information that includes information on nodes respectively representing basic constituent elements of a metabolism and edges respectively representing reaction relationships between the basic constituent elements;
an environmental factor information setting step of allowing the user to set environmental factor information on an environmental factor that influences both of or one of the basic constituent elements and the reaction relationships;
an end condition information setting step of allowing the user to set end condition information on a condition for finishing a simulation;
a simulation execution step of changing both of or one of one of the nodes and one of the edges included in the target metabolic circuit information, and of executing a metabolic simulation by a metabolic flux balance analysis using the environmental factor information set at the environmental factor information setting step;
a simulation result determination step of determining whether a result of the simulation executed at the simulation execution step satisfies the end condition information set at the end condition information setting step, and of, if the result of the simulation does not satisfy the end condition information, changing both of or one of another one of the nodes and another one of the edges, followed by execution of the simulation execution step; and
a simulation result output step of outputting the result of the simulation executed at the simulation execution step.

**16.** The program according to claim 15, wherein each of the basic constituent elements includes information on at least one of a substrate, a metabolite, and a matter in vivo.

**17.** The program according to claim 15, wherein each of the reaction relationships includes information on a relationship of at least one of an enzyme reaction, a transcription control reaction, a translation control reaction, and a chemical reaction between the basic constituent elements.

**18.** The program according to claim 15, wherein the environmental factor information includes information on at least one of a temperature, a pH, an atmospheric pressure, the number of times of stirring, a dissolved oxygen quantity, a culture medium component, an optical condition, and a carbon dioxide concentration.

**19.** The program according to claim 15, wherein the end condition information includes information on at least one of a culture time, a metabolic efficiency, a metabolite quantity, the number of times of simulation, a cell density, a culture cost, a pH, a dissolved oxygen quantity, and a culture medium component quantity.

**20.** The program according to claim 15, further comprising:

a mutation frequency information storage unit of storing mutation frequency information on a frequency with which both of or one of the one node and the one edge included in the metabolic circuit information mutates to both of or one of another one of the nodes and another one of the edges, wherein
at the simulation execution unit, both of or one of the one node and the one edge included in the metabolic circuit information is changed using the mutation frequency information stored at the mutation frequency information storage unit.

21. The program according to claim 15, wherein at the simulation execution unit, both of or one of the one node and the one edge included in the metabolic circuit information is changed using at least one of a genetic algorithm and simulated annealing.

22. A computer readable recording medium that records the program according to any one of claims 15 to 21.

EXPLANATORY VIEW FOR PRINCIPLE OF PRESENT INVENTION

FIG.1

EP 1 449 926 A1

# FIG.2

INPUT DEVICE — 112

OUTPUT DEVICE — 114

METABOLIC CIRCUIT INFORMATION PROCESSING APPARATUS — 100

INPUT AND OUTPUT CONTROL IF UNIT — 108

EXTERNAL SYSTEM — 200

EXTERNAL DB

EXTERNAL ANALYSIS PROGRAM

STORAGE UNIT — 106

METABOLIC CIRCUIT INFORMATION DB — 106a

ENVIRONMENTAL FACTOR INFORMATION DB — 106b

END CONDITION INFORMATION DB — 106c

MUTATION DB — 106d

CONTROL UNIT — 102

DATABASE CREATION UNIT — 102a

SIMULATION CONDITION SETTING UNIT — 102b

SIMULATION UNIT — 102c

COMMUNICATION CONTROL IF UNIT — 104

300

EP 1 449 926 A1

# FIG.3

METABOLIC CIRCUIT INFORMATION DATABASE
106a

| ORGANISM NAME | |
|---|---|
| CELL NAME | |
| METABOLITE NAME | |

| NODE INFORMATION | | EDGE INFORMATION | | |
|---|---|---|---|---|
| NODE NAME | ADDITIONAL INFORMATION | CONNE-CTION SOURCE | CONNE-CTION DESTIN-ATION | ADDITIONAL INFORMATION |
| | | | | |
| | | | | |
| ⋮ | ⋮ | ⋮ | ⋮ | ⋮ |

# FIG.4

ENVIRONMENTAL FACTOR INFORMATION DATABASE

106b

| TEMPER-ATURE | pH | ATMOSPHERIC PRESSURE | NUMBER OF TIMES OF STIRRING | DISSOLVED OXYGEN QUANTITY | CULTURE MEDIUM COMPONENT | OPTICAL CONDITION | CARBON DIOXIDE CONCENTRATION | ⋯ |
|---|---|---|---|---|---|---|---|---|
| | | | | | | | | ⋯ |
| | | | | | | | | ⋯ |
| ⋮ | ⋮ | ⋮ | ⋮ | ⋮ | ⋮ | ⋮ | ⋮ | ⋯ |

EP 1 449 926 A1

# FIG.5

END CONDITION INFORMATION DATABASE
/106c

| CULTURE TIME | METABOLIC EFFICIENCY | METABOLITE QUANTITY | NUMBER OF TIMES OF SIMULATION | CELL DENSITY | CULTURE COST | pH | DISSOLVED OXYGEN QUANTITY | CULTURE MEDIUM COMPONENT QUANTITY | ... |
|---|---|---|---|---|---|---|---|---|---|
|  |  |  |  |  |  |  |  |  | ... |
|  |  |  |  |  |  |  |  |  | ... |
| : | : | : | : | : | : | : | : | : | ... |

EP 1 449 926 A1

EP 1 449 926 A1

# FIG.6

DATABASE CREATION UNIT
102a

| METABOLIC CIRCUIT INFORMATION DB CREATION UNIT | — 102d |
| ENVIRONMENTAL FACTOR INFORMATION DB CREATION UNIT | — 102e |
| END CONDITION INFORMATION DB CREATION UNIT | — 102f |

27

# FIG.7

SIMULATION CONDITION SETTING UNIT
102b

METABOLIC CIRCUIT
SETTING UNIT — 102g

ENVIRONMENTAL FACTOR
SETTING UNIT — 102h

END CONDITION SETTING UNIT — 102i

# FIG.8

(SYSTEM MAIN PROCESSING)

```
        ┌─────────────┐
        │    START     │
        └─────────────┘
               │
               ▼
┌──────────────────────────────────────────┐
│  EXECUTE DATABASE CREATION PROCESSING     │──SA-1
└──────────────────────────────────────────┘
               │
               ▼
┌──────────────────────────────────────────┐
│       EXECUTE SIMULATION CONDITION        │──SA-2
│            SETTING PROCESSING             │
└──────────────────────────────────────────┘
               │
               ▼
┌──────────────────────────────────────────┐
│      EXECUTE SIMULATION PROCESSING        │──SA-3
└──────────────────────────────────────────┘
               │
               ▼
        ┌─────────────┐
        │     END      │
        └─────────────┘
```

29

# FIG.9

(DB CREATION PROCESSING)

```
        ┌─────────────┐
        │    START    │
        └──────┬──────┘
               │
               ▼
   ┌───────────────────────────┐
   │  EXECUTE METABOLIC CIRCUIT │
   │  INFORMATION DB CREATION   │─── SB-1
   │  PROCESSING                │
   └──────────────┬────────────┘
                  │
                  ▼
   ┌───────────────────────────┐
   │  EXECUTE ENVIRONMENTAL     │
   │  FACTOR INFORMATION DB     │─── SB-2
   │  CREATION PROCESSING       │
   └──────────────┬────────────┘
                  │
                  ▼
   ┌───────────────────────────┐
   │  EXECUTE END CONDITION     │
   │  INFORMATION DB CREATION   │─── SB-3
   │  PROCESSING                │
   └──────────────┬────────────┘
                  │
                  ▼
        ┌─────────────┐
        │     END     │
        └─────────────┘
```

# FIG.10

## (METABOLIC CIRCUIT INFORMATION DB CREATION PROCESSING)

START

ACCESS EXTERNAL DB — SC-1

ACQUIRE METABOLIC CIRCUIT INFORMATION FROM EXTERNAL SYSTEM — SC-2

STORE INFORMATION IN METABOLIC CIRCUIT INFORMATION DB — SC-3

END

# FIG.11

(ENVIRONMENTAL FACTOR
INFORMATION DB CREATION PROCESSING)

START

ACCESS METABOLIC CIRCUIT
INFORMATION DB — SD-1

EXTRACT ENVIRONMENTAL
FACTOR INFORMATION — SD-2

ALLOW USER TO ADDITIONALLY
SET OTHER ENVIRONMENTAL
FACTOR INFORMATION — SD-3

STORE INFORMATION IN
ENVIRONMENTAL FACTOR
INFORMATION DB — SD-4

END

# FIG.12

(END CONDITION INFORMATION DB CREATION PROCESSING)

```
        ┌─────────────┐
        │    START    │
        └─────────────┘
               │
               ▼
┌─────────────────────────────┐
│   ACCESS METABOLIC CIRCUIT   │── SE-1
│       INFORMATION DB         │
└─────────────────────────────┘
               │
               ▼
┌─────────────────────────────┐
│  EXTRACT DATA ON END CONDITION │── SE-2
└─────────────────────────────┘
               │
               ▼
┌─────────────────────────────┐
│   ALLOW USER TO ADDITIONALLY │
│   SET OTHER END CONDITION    │── SE-3
│        INFORMATION           │
└─────────────────────────────┘
               │
               ▼
┌─────────────────────────────┐
│   STORE INFORMATION IN END   │── SE-4
│  CONDITION INFORMATION DB    │
└─────────────────────────────┘
               │
               ▼
        ┌─────────────┐
        │     END     │
        └─────────────┘
```

# FIG.13

(SIMULATION CONDITION SETTING PROCESSING)

```
        ┌─────────────────┐
        │      START       │
        └─────────────────┘
                 │
                 ▼
   ┌─────────────────────────────┐
   │  ALLOW USER TO SET METABOLIC │  SF-1
   │      CIRCUIT INFORMATION     │
   └─────────────────────────────┘
                 │
                 ▼
   ┌─────────────────────────────┐
   │   ACQUIRE USER'S SET METABOLIC│
   │     CIRCUIT INFORMATION FROM  │  SF-2
   │  METABOLIC CIRCUIT INFORMATION DB│
   └─────────────────────────────┘
                 │
                 ▼
   ┌─────────────────────────────┐
   │ ALLOW USER TO SET ENVIRONMENTAL│  SF-3
   │       FACTOR INFORMATION      │
   └─────────────────────────────┘
                 │
                 ▼
   ┌─────────────────────────────┐
   │  ALLOW USER TO SET END CONDITION│  SF-4
   │          INFORMATION          │
   └─────────────────────────────┘
                 │
                 ▼
        ┌─────────────────┐
        │       END        │
        └─────────────────┘
```

# FIG.14

(METABOLIC CIRCUIT SETTING SCREEN)

· ORGANISM NAME/CELL NAME    MA-1      ( REFERENCE ) MA-2

· METABOLIC CIRCUIT NAME    MA-3      ( REFERENCE ) MA-4

· METABOLITE NAME    MA-5      ( REFERENCE ) MA-6

SETTING END    MA-7

# FIG.15

(ENVIRONMENTAL FACTOR SETTING SCREEN)

ENVIRONMENTAL 1.
FACTOR
MB-1

2.
MB-2

3.
MB-3

4.
MB-4

MB-5

SETTING END
MB-6

# FIG.16

(END CONDITION SETTING SCREEN)

CULTURE TIME        ▭ — MC-1

METABOLIC
EFFICIENCY        ▭ — MC-2

NUMBER OF TIMES
OF SIMULATION        ▭ — MC-3

.
.
.

SETTING END — MC-4

# FIG.17

(SIMULATION PROCESSING)

```
        ┌──────────────┐
        │    START     │
        └──────┬───────┘
               │
        ┌──────▼───────────┐
        │ ACCESS MUTATION DB│  ─ SG-1
        └──────┬───────────┘
               │
        ┌──────▼───────────────┐
        │  ACQUIRE MUTATION     │  ─ SG-2
        │ FREQUENCY INFORMATION │
        └──────┬───────────────┘
               │
        ┌──────▼───────────────┐
        │ PERFORM BOTH OF OR    │
        │   ONE OF KI PROCESSING│  ─ SG-3
        │   AND KO PROCESSING   │
        └──────┬───────────────┘
               │
        ┌──────▼───────────────┐
        │     PERFORM FBA       │  ─ SG-4
        └──────┬───────────────┘
               │
           ◇ DETERMINE WHETHER          SG-5
      No ◇   SOLUTION SATISFIES
         ◇   END CONDITIONS
               │ Yes
        ┌──────▼───────────────┐
        │ EDIT SOLUTION, AND    │
        │ CREATE SIMULATION     │  ─ SG-6
        │ RESULT SCREEN DATA    │
        └──────┬───────────────┘
               │
        ┌──────▼───────────────┐
        │ OUTPUT SIMULATION     │  ─ SG-7
        │ RESULT SCREEN DATA    │
        └──────┬───────────────┘
               │
        ┌──────▼───────┐
        │     END      │
        └──────────────┘
```

# FIG.18

EP 1 449 926 A1

# FIG.19

SIMULATION RESULT 1

| INDIVIDUAL No. | CIRCUIT DIAGRAM | METABOLIC EFFICIENCY |
|---|---|---|
| 1 | [E KNOCK-OUT] | 1.51 |
| 2 | [H INSERT] | 1.25 |
| ⋮ | ⋮ | ⋮ |

MD-1   MD-2   MD-3   MD-4   MD-5   MD-6   MD-7

# FIG.20

SIMULATION RESULT 2

ME-1

ENVIRONMENTAL FACTORS

1 . TEMPERATURE [              ] ME-2

2.  pH [              ] ME-3

⋮   ⋮                  ⋮

CULTURE TIME [              ] ME-4

METABOLIC
EFFICIENCY [              ] ME-5

⋮                  ⋮

# INTERNATIONAL SEARCH REPORT

| | International application No. |
|---|---|
| | PCT/JP02/13012 |

| A. CLASSIFICATION OF SUBJECT MATTER |
|---|
| Int.Cl$^7$ C12Q3/00, G06F19/00 |

According to International Patent Classification (IPC) or to both national classification and IPC

| B. FIELDS SEARCHED |
|---|
| Minimum documentation searched (classification system followed by classification symbols) |
| Int.Cl$^7$ C12Q3/00, G06F19/00 |

Documentation searched other than minimum documentation to the extent that such documents are included in the fields searched

Electronic data base consulted during the international search (name of data base and, where practicable, search terms used)
JICST FILE(JOIS), WPI(DIALOG)

## C. DOCUMENTS CONSIDERED TO BE RELEVANT

| Category* | Citation of document, with indication, where appropriate, of the relevant passages | Relevant to claim No. |
|---|---|---|
| A | Jeremy S. EDWARDS et al., Systems Properties of the Haemophilus influenzae Rd Metabolic Genotype. THE JOURNAL OF BIOLOGICAL CHEMISTRY, June 1999, Vol.274, No.25, pages 17410 to 17416 | 1-22 |
| A | Neema JAMSHIDI et al., Dynamic simulation of the human red blood metabolic network. BIOINFORMATICS 2001, Vol.17, No.3, pages 286 to 287 | 1-22 |
| A | Jeremy S. EDWARDS et al., In silico predictions of Eschericchia coli metabolic capabilities are consistent with experimental data. NATURE BIOTECHNOLOGY, February 2001, Vol.19, pages 125 to 130 | 1-22 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 20 February, 2003 (20.02.03) | 04 March, 2003 (04.03.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

International application No.

PCT/JP02/13012

C (Continuation). DOCUMENTS CONSIDERED TO BE RELEVANT

| Category* | Citation of document, with indication, where appropriate, of the relevant passages | Relevant to claim No. |
|---|---|---|
| A | Christophe H. SCHILLING et al., The underlying pathway structure of biochemical reaction network. Proc.Natl.Acad.Sci.USA, April 1998, Vol.95, pages 4193 to 4198 | 1-22 |

Form PCT/ISA/210 (continuation of second sheet) (July 1998)